# EUROPÄISCHE PATENTSCHRIFT

(11) **EP 3 933 236 B1**
(45) Veröffentlichungstag und Bekanntmachung des Hinweises auf die Patenterteilung: **14.02.2024**
(21) Anmeldenummer: 21179935.8
(22) Anmeldetag: 17.06.2021
(51) Int. Cl.: F16K 11/052, A61M 16/22, F15C 3/04, F16K 31/12, F16K 31/165, F16K 31/128, A61M 16/20, A61M 16/08

(54) **PNEUMATISCH BETÄTIGTES SCHALTVENTIL, VENTILANORDNUNG UND BEATMUNGSSYSTEM**
PNEUMATICALLY ACTUATED SWITCHING VALVE, VALVE ASSEMBLY AND VENTILATION SYSTEM
SOUPAPE DE COMMANDE PNEUMATIQUE, AGENCEMENT DE SOUPAPE ET SYSTÈME RESPIRATOIRE

(30) Priorität: 01.07.2020 DE 102020117374
(43) Veröffentlichungstag der Anmeldung: 05.01.2022
(73) Patentinhaber: Samson Aktiengesellschaft, 60314 Frankfurt am Main (DE)
(72) Erfinder: Pittelkow, Michael, 63500 Seligenstadt (DE)
(74) Vertreter: Schmid, Nils T.F.

(56) Entgegenhaltungen:
- EP-A1- 3 418 586
- DE-A1- 2 204 456
- DE-B- 1 301 724
- DE-B1- 2 534 139
- DE-B3-102011 107 494
- FR-A- 1 480 927
- FR-A1- 2 711 923
- US-A- 5 537 995

## Beschreibung

Die Erfindung betrifft ein pneumatisch betätigtes Schaltventil. Das pneumatisch betätigte Schaltventil kann beispielsweise in einem therapeutischen Beatmungssystem zur Behandlung von Menschen oder Tieren eingesetzt werden. Andere denkbare Anwendungsgebiete umfassen die Verwendung eines pneumatisch betätigten Schaltventils in einer prozesstechnischen Anlage, wie einer lebensmittelverarbeitenden Anlage, beispielsweise einer Brauerei, ein Kraftwerk, einer chemischen Anlage, wie einer petrochemischen Anlage, oder dergleichen. Die Erfindung betrifft auch eine Ventilanordnung, die zwei pneumatisch betätigte Schaltventile umfasst. Pneumatisch betätigte Schaltventile und durch sie gebildete Ventilanordnungen können eingesetzt werden in Anlagen oder Vorrichtungen, bei denen es unerwünscht oder unvorteilhaft ist, zusätzlich eine beispielsweise elektrische Hilfsenergie zum Steuern und/oder Antreiben zu verwenden, wie beispielsweise in einem entzündungs- oder explosionsgefährdeten Bereich. Die Erfindung betrifft auch ein Beatmungssystem mit wenigstens einem pneumatisch betätigten Schaltventil oder einer Ventilanordnung aus zwei oder mehreren pneumatisch betätigten Schaltventilen.

Pneumatisch betätigte Schaltventile sind weithin verbreitet dank ihres strukturell einfachen Aufbaus, der sich in geringen Herstellkosten, guter Wartungsfreundlichkeit, hoher Verlässlichkeit und Betriebssicherheit niederschlägt. Ein gattungsgemäßes pneumatisch betätigtes Schaltventil wird im Folgenden unter Verweis auf DE 25 34 139 B1 erläutert. Das pneumatisch betätigte Schaltventil ist gebildet durch ein Schaltergehäuse mit zwei schalenartigen Gehäusehälften, in deren Trennebene eine elastische Steuermembran angeordnet ist. Die Steuermembran bildet im Schaltergehäuse zwei Steuerkammern, die durch die Membran voneinander getrennt sind. Die Steuermembran ist integral mit einem Betätigungshebel verbunden, der an seinem steuerkammer-abgewandten Ende einen Ventilkörper mit zwei einander gegenüberliegenden Dichtflächen bildet. Je nach Beaufschlagung einer der Steuerkammern mit Druckluft über Steuerluftkanäle wird der Ventilkörper entweder in einem ersten Ventilsitz mit einem ersten Druckmittelkanal oder einem zweiten Ventilsitz eines zweiten Druckmittelkanals zur Anlage gebracht, wodurch eine Arbeitsfluid- oder Druckmittel-Verbindung zwischen einem Druckmittelauslasskanal, der immer mit der Schaltventilkammer in Verbindung steht und dem ersten oder zweiten Druckmittelkanal hergestellt wird. Einer der Druckmittelkanäle kann eine Belüftungsöffnung sein. Derartige pneumatisch betätigte Schaltventil werden auch in DE 1 301 724 A und in DE 1857 693 U beschrieben.

Eine Ventilanordnung, die sich pneumatisch betätigten Schaltventilen bedient, wird in DE 22 04 456 B2 beschrieben. Die Ventilanordnung umfasst mehrere pneumatische Schaltventile, die mit einander über Pneumatikleitungen verbunden sind, um ein statisches Speicherelement als binäre pneumatische Schaltlogik zu bilden. Bei der Ventilanordnung wird ein Versorgungsfluid einem Fluideinlass zugeführt, welcher in einer Ruhestellung eines 3-Wegeventils offen ist. Ein erstes 3-Wegeventil hat einem Fluideinlass und einen federbelasteten Steuereingang als logische Setzeingänge. Ein zweites 3-Wegeventil weist seinem Fluideinlass und einem federbelasteten Steuereingang logische Löscheingänge auf. Das so gebildete pneumatische Speicherelement weist die Form einer bistabilen Kippstufe auf.

US 5,537,995 A beschreibt ein geschlossenes oder halbgeschlossenes Beatmungssystem zum Schutz vor Brandrauch, das einen pneumatisch gesteuerten Beatmungsbeutel in Verbindung mit einem Atemstück (etwa einem Mundstück oder einer Atemschutzmaske) zum Tragen im Gesicht des Nutzers, einem Mittel zum Absorbieren von ausgeatmeten CO₂, einem pneumatischen Aktuator zum abwechselnden Expandieren und Komprimieren des Beatmungsbeutels entsprechend dem Atemrhythmus des Nutzers und einer damit verbundenen Druckluftquelle umfasst.

Bekannte pneumatisch betätigte Schaltventile und Ventilanordnungen lassen sich für eine Vielzahl unterschiedlicher Anwendung einsetzen. Abhängig von dem jeweiligen konkreten Anwendungsfall wird ein pneumatisch betätigtes Stellventil mit den jeweils erforderlichen pneumatischen Versorgungsleitungen und Belüftungsleitungen ausgestattet. Eine gesteuerte und/oder geregelte Druckluftbeaufschlagung der Steuerkammer wird sodann bereitgestellt, um die gewünschte Schaltfunktion des 3/2-Wegeventil zu bewirken. Die voneinander unabhängigen Druckversorgungsleitungen für unterschiedliche Drucksignale oder Arbeitsdrucke können in einigen Fällen komplexe Aufbauten erfordern. Für einzelne Anwendungszwecke kann zur Verwendung eines pneumatisch betätigten Schaltventils oder einer Ventilanordnung mit einem oder mehreren pneumatisch betätigten Schaltventilen mitunter eine komplexe pneumatische Verschaltung erforderlich werden. Komplexe Verschaltungen negieren jedoch die grundsätzlichen Vorteile von pneumatisch betätigten Schaltventilen.

Es kann als eine Aufgabe der Erfindung gesehen werden, die Nachteile des Stands der Technik zu überwinden und ein pneumatisch betätigtes Schaltventil und/oder eine Ventilanordnung bereitzustellen, das/die die Nachteile des Stands der Technik überwindet und insbesondere ohne komplizierte Verschaltung einen pneumatischen Arbeitsdruck in einem periodischen, insbesondere zyklischen, Verlauf bereitstellt, beispielsweise zur therapeutischen Unterstützung der Respiration. Diese Aufgabe lösen die Gegenstände der unabhängigen Ansprüche.

Demnach ist ein pneumatische betätigtes Schaltventil vorgesehen, das eine Schaltventilkammer aufweist, in die drei Luftkanäle münden, wobei die drei Luftkanäle einen Luftversorgungskanal zum Empfangen von Druckluft von einer Druckluftversorgungsquelle, einem Hauptluftkanal zum Be- und Entlüften wenigstens einer anderen pneumatischen Komponente und einen Entlüftungskanal zum Abgeben von Druckluft an eine Drucksenke, wie die Atmosphäre, umfassen. In der Schaltventilkammer ist ein Schaltventilglied angeordnet, um den Luftversorgungskanal oder den Entlüftungskanal zu verschließen. Das Schaltventilglied ist beweglich zwischen einer ersten Schließstellung, in der das Schaltventilglied den Luftversorgungskanal verschließt, und einer zweiten Schließstellung, in der das Schaltventilglied den Entlüftungskanal verschließt. Der Hauptluftkanal ist stets fluidleitend mit der Schaltventilkammer verbunden. Die Öffnungsweite des Hauptluftkanals ist von der Stellung des Schaltventilgliedes unabhängig. Die Schaltventilkammer mit dem Luftversorgungskanal, dem Hauptluftkanal, dem Entlüftungskanal und dem Schaltventilglied kann als sogenanntes 3/2-Wegeventil bezeichnet sein. Abhängig von der Stellung des Schaltventilgliedes in der Schaltventilkammer ist der Hauptluftkanal fluidisch verbunden mit dem Luftversorgungskanal und/oder dem Entlüftungskanal. Das Schaltventilglied kann gegebenenfalls eine Zwischenstellung zwischen der ersten Schließstellung und der zweiten Schließstellung einnehmen, in der das Schaltventilglied keinen der drei Luftkanäle der Schaltventilkammer verschließt. Dies kann für einige Anwendungen nützlich sein. Gemäß einer alternativen Ausführung ist das Schaltventilglied derart konfiguriert, dass stets entweder der Luftversorgungskanal oder der Entlüftungskanal verschlossen ist, sodass durch die Schaltventilkammer ausschließlich entweder Luftversorgungskanal und Hauptluftkanal oder ausschließlich Entlüftungskanal und Hauptluftkanal miteinander verbunden sind.

Gattungsgemäß weist das pneumatisch betätigte Schaltventil ferner zur pneumatischen Betätigung des Schaltventilgliedes einen pneumatischen Aktor auf. Der pneumatische Aktor des pneumatisch betätigten Schaltventils ist pneumatisch unterteilt in eine Betätigungskammer und eine Rückstellkammer, wobei ein erster Steuerdruck in der Betätigungskammer entgegen einer Federrückstellung und entgegen einem zweiten Steuerdruck in der Rückstellkammer wirkt. Die Betätigungskammer kann einen Stelldruckkanal zum Empfangen des ersten Steuerdrucks aufweisen. Die Rückstellfeder ist vorzugsweise in der Rückstellkammer angeordnet. Alternativ oder zusätzlich kann eine Rückstellfeder in der Betätigungskammer angeordnet sein. Die Rückstellfeder kann gemäß einer ersten Ausführung eine Vorspannung in Richtung der ersten Schließstellung zu bewirken. Gemäß dieser ersten Ausführung kann mit einem ersten Steuerdruck in der Steuerkammer bei überschreiten eines Schalt-Schwelldruckwerts ein verlagern des Schaltventilglieds in die zweite Schließstellung bewirkt werden. Die Rückstellfeder kann gemäß einer zweiten Ausführung eine Vorspannung in Richtung der zweiten Schließstellung zu bewirken. Gemäß dieser zweiten Ausführung kann mit einem ersten Steuerdruck in der Steuerkammer bei überschreiten eines Schalt-Schwelldruckwerts ein verlagern des Schaltventilglieds in die erste Schließstellung bewirkt werden. Gemäß einer Ausführung ist es denkbar, dass das Trennglied, welches die Betätigungskammer und die Rückstellkammer pneumatisch voneinander separiert, dazu ausgestaltet ist, eine Federrückstellung entgegen dem ersten Steuerdruck in der Betätigungskammer bereitzustellen. Das Trennglied ist eine Membrane oder ein Balg. Das pneumatisch betätigte Schaltventil ist insbesondere derart ausgestaltet, dass die Betätigungskammer mit Ausnahme ihres Stelldruckkanals luftdicht abgeschlossen ist, insbesondere gegenüber der Druckluftversorgungsquelle und/oder der Drucksenke und/oder der Rückstellkammer, vorzugsweise für Druckdifferenzen zwischen dem Druck der Druckluftversorgungsquelle und dem der Drucksenke, insbesondere der Atmosphäre, bis 2 bar, bis zu 5 bar oder sogar bis zu 10 bar.

Erfindungsgemäß ist vorgesehen, dass die Rückstellkammer einen Rückstelldruckkanal zum Bereitstellen von Druckluft an die Rückstellkammer aufweist sowie einen mit einer Auslassdrossel ausgestatteten Staudruckkanal zum Abgeben von Druckluft aus der Rückstellkammer an eine Drucksenke, wie die Atmosphäre. Insbesondere ist der Rückstelldruckkanal ein strukturell von dem Staudruckkanal unterschiedlicher Kanal ist. Die Rückstellkammer ist insbesondere mit Ausnahme des Staudruckkanals einerseits und des Rückstelldruckkanals andererseits luftdicht abgeschlossen, insbesondere gegenüber der Druckluftversorgungsquelle und/oder der Drucksenke und/oder der Betätigungskammer, vorzugsweise für Druckdifferenzen zwischen dem Druck der Druckluftversorgungsquelle und dem der Drucksenke, insbesondere der Atmosphäre, bis zu 2 bar, bis zu 5 bar oder sogar bis zu 10 bar.

Die Ausgestaltung der Rückstellkammer mit einem Staudruckkanal erlaubt für einige Anwendungen des pneumatisch betätigten Stellventils die Verwendung einer geringeren Anzahl von Druckversorgungskanälen, indem ein in dem System anderweitig vorhanden Pneumatikdruck für die Bereitstellung des zweiten Steuerdrucks in der Rückstellkammer hergenommen wird. Auf diese Weise lässt sich der Verschaltungsaufwand und die erforderliche Anzahl an Versorgungsanschlüsse reduzieren. Es sei klar, dass das pneumatisch betätigte Schaltventil frei ist von elektrischen Antriebsmitteln, wie einem Elektromotor, und/oder frei ist von elektrischen Schaltmitteln, wie einem Solenoidventil, insbesondere frei von Elektronik.

Gemäß einer Ausführung der Erfindung, die mit den vorherigen kombinierbar ist, ist das Schaltventilglied einstückig gebildet mit einem die Betätigungskammer und die Rückstellkammer pneumatisch trennenden Trennglied, insbesondere einer Membran. Beispielsweise kann das Trennglied gemeinsam mit dem Schaltventilglied in der Schaltkammer als einteiliger, vorzugsweise faserverstärkter, Kunststoffkörper, insbesondere Elastomerkörper, gebildet sein. Eine Verformung der Membran durch eine bestimmte aufeinander abgestimmte Einstellung des ersten Steuerdrucks und des zweiten Steuerdrucks bewirkt eine Einstellung des Schaltventilglieds in dessen erster oder zweiter Schließstellung oder gegebenenfalls einer dazwischen liegenden Zwischenstellung. Es ist denkbar, dass das bewegliche und/oder insbesondere elastisch verformbare Trennglied verbunden ist mit einem Schaltventilglied, das beispielsweise als Platte, beispielsweise aus Kunststoff und/oder Metall, gebildet sein kann. Das Schaltventilglied und das Trennglied können über ein Gelenk, insbesondere ein Kippgelenk und/oder ein Festkörpergelenk, miteinander verbunden sein.

Gemäß einer bevorzugten Ausführung eines erfindungsgemäßen pneumatisch betätigten Schaltventils ist der Rückstelldruckkanal fluidisch verbunden mit dem Hauptluftkanal und/oder dem Luftversorgungskanal. Alternativ oder zusätzlich verbindet der Rückstelldruckkanal die Schaltventilkammer mit der Rückstellkammer. Vermittels des Rückstelldruckkanals kann in der Rückstellkammer der zweite Steuerdruck bereitgestellt werden, welcher bewirkt wird mithilfe eines in dem Schaltventil an anderer Stelle vorhandenem Pneumatikfluid. Insbesondere wird das Arbeitsfluid, welches mithilfe des pneumatisch betätigten Schaltventils eingestellt wird und bei Betrieb in der Schaltventilkammer zur Verfügung steht, verwendet, um den zweiten Steuerduck zu bewirken. Anstelle einer konventionell üblichen, zusätzlichen, nur für die zweite Steuerkammer bzw. Rückstellkammer vorhandenen Hilfsfluid-Quelle nebst dazugehöriger Verschaltung sowie gegebenenfalls erforderlicher Elektronik kann durch die Verwendung des Rückstelldruckkanals eine erhebliche Vereinfachung des Aufbaus bewirkt werden. Vorzugsweise münden in die Schaltventilkammer nicht mehr als die drei Luftkanäle (Luftversorgungskanal, Hauptluftkanal und Entlüftungskanal) sowie gegebenenfalls der Rückstelldruckkanal. Insbesondere ist das pneumatisch betätigte Schaltventil derart ausgestaltet, dass die Schaltventilkammer mit Ausnahme der Luftkanäle und des Rückstelldruckkanal luftdicht abgeschlossen ist, insbesondere gegenüber der Druckluftversorgungsquelle und/oder der Drucksenke, vorzugsweise für Druckdifferenzen zwischen dem Druck der Druckluftversorgungsquelle und dem Druck der Drucksenke, insbesondere der Atmosphäre, bis zu 2 bar, bis zu 5 bar oder bis zu 10 bar.

Während bei zahlreichen konventionellen pneumatischen betätigten Schaltventilen kein vom Atmosphärendruck abweichender zweiter Steuerdruck bereitgestellt wird, sodass entgegen dem ersten Steuerdruck der Betätigungskammer nur die Federrückstellung wirkt, erlaubt es die Kombination der Rückstellwirkung einer Rückstellfeder und eines zweiten Steuerdrucks, die Schaltwirkung des Schaltventils zu modifizieren und gegebenenfalls variabel einstellbar zu gestalten.

Gemäß einer anderen Ausführung, die mit der vorherigen kombinierbar ist, ist in dem Rückstelldruckkanal eine Steuerdrossel zum Einstellen des zweiten Steuerdrucks angeordnet. Die Steuerdrossel bewirkt, dass der zweite Steuerdruck in der stromabwärts der Steuerdrossel liegenden, über den Staudruckkanal belüfteten, Rückstellkammer niedriger ist, als in einem Abschnitt des Rückstelldruckkanals und/oder einem vorgeschalteten Druckversorgungskanal stromaufwärts der Steuerdrossel. Die Steuerdrossel kann einen konstanten Drosselquerschnitt haben. Die Steuerdrossel kann aus einem statischen, ortsfesten Drosselglied bestehen. Das statische Drosselglied weist eine konstante Drosselwirkung auf. In vielen Fällen kann es wünschenswert sein, einen Arbeitsdruck oder einen Steuerdruck aus einem Teil des pneumatischen Systems des pneumatisch betätigten Schaltventils oder einer Ventilanordnung ab zu greifen, wo ein wesentlich höherer lokaler pneumatischer Druck herrscht, als der für die korrekte bzw. gewünschte Betätigung des Aktors erforderliche zweiter Steuerdruck. Die Verwendung des der Steuerdrossel erlaubt die Bereitstellung eines zweiten Steuerdrucks an die Rückstellkammer, welcher sich auf bestimmten Weise unterscheidet von dem Speisedruck an der Stelle des pneumatischen Systems, von der ausgehend der Rückstelldruckkanal abzweigt.

Gemäß einer Weiterbildung der Erfindung weist die Steuerdrossel eine variable Drosselweite auf. Die Drosselweite der Steuerdrossel kann beispielsweise in Querschnitts- und/oder in Axialrichtung variabel sein. Beispielsweise kann eine Steuerdrossel einen veränderlichen Drosselquerschnitt aufweisen. Alternativ oder zusätzlich ist es denkbar, dass die Steuerdrossel einen ortsfesten Sitz und ein insbesondere ein Axialrichtung bewegliche Drosselglied aufweist, die gemeinsam eine Drosselweite festlegen, wobei das bewegliche Drosselglied auf den Sitz zu und/oder von dem Sitz weg verfahrbar ist. Insbesondere besteht die Steuerdrossel aus einem statischen Steuerdrosselglied und einem beweglichen Steuerdrosselglied. Zusätzlich oder alternativ kann das bewegliche Drosselglied in den Sitz hinein und/oder aus dem Sitz hinaus verfahrbar sein. Insbesondere weist die Steuerdrossel ein bewegliches Drosselglied auf, wie eine Madenschraube. Vorzugsweise kann die Steuerdrossel ein ortfestes Drosselglied, wie einen insbesondere ringförmigen Sitz, mit konstantem Drosselquerschnitt umfassen. Gemäß einer anderen Ausführung umfasst die Steuerdrossel ein ortsfestes Drosselglied und ein bewegliches Drosselglied, die relativ zueinander beweglich sind, um die Drosselweite einzustellen. Die Drosselweite kann diskret oder kontinuierlich einstellbar sein. Eine diskrete Einstellbarkeit der Steuerdrossel kann beispielsweise dadurch gewährleistet sein, dass ein Teil der Drossel als Lochblende ausgeführt ist der andere Teil als Lochblendenabdeckung, wobei die Lochblende und die Lochblendenabdeckung relativ zueinander verfahrbar ist. Abhängig von einer Relativstellung von Lochblende und Lochblendenabdeckung zueinander kann eine unterschiedliche Anzahl von Löchern verdeckt und/oder offengelegt sein.

Gemäß einer bevorzugten Ausführung ist ein statisches, ortsfestes Drosselglied als ringförmige Komponente gebildet, die eine Querschnittsflächen des Rückstelldruckkanals bildet und ein bewegliches Drosselglied ist als Madenschraube gebildet, die in Axialrichtung beweglich ist, um eine Drosselweite zwischen dem ortsfesten oder dem beweglichen Drosselglied zu variieren und dadurch eine Druckdifferenz einstellen zu können. Die Steuerdrossel variabler Drosselweite erlaubt eine hochpräzise Einstellung des Rückstelldruckkanals, um eine präzise Schaltfunktion des pneumatisch betätigten Schaltventils mit schmalem Toleranzbereich zu erreichen. Auf diese Weise lässt sich ein pneumatisch betätigtes Schaltventil hoher Präzision besonders kostengünstig bereitstellen. Ein derartiges präzise einstellbares pneumatisch betätigtes Schaltventil eignet sich besonders gut zur Kombination mit einem oder mehreren weiteren pneumatisch betätigten Schaltventilen gleicher oder anderer Bauart in einer Ventilanordnung, wo pneumatische Toleranzen mehrerer Schaltventilen einander verstärken. Nicht variabel einstellbare Steuerdrosseln müssten in Ventilanordnungen zum erreichen schmaler Toleranzbänder nicht bloß aufwändig gefertigt werden, sondern individuell paarweise aufeinander abgestimmt selektiert werden, wodurch sich die Bereitstellungskosten stark erhöhen und was die Wartbarkeit enorm erschweren würde.

Gemäß einer Ausführung kann das pneumatisch betätigte Schaltventil mit einstellbarer Federvorspannung ausgeführt sein. Insbesondere kann eine Federvorspannung, ein Federweg und/oder eine Federkonstante einer Rückstellfeder des Aktors einstellbar sein. Die Rückstellfeder umfasst insbesondere ein Stellmittel, um einen unteren Mindestschwellwert der Rückstellkraft einzustellen, welche wenigstens entgegen dem ersten Steuerdruck in der Betätigungskammer wirkt.

Gemäß einer Ausführung der Erfindung, die mit den vorherigen kombinierbar ist, sind der pneumatische Aktor und die Schaltventilkammer in demselben Gehäuse untergebracht. Insbesondere ist der Rückstelldruckkanal durch das Gehäuse gebildet. Insbesondere ist die Rückstellfeder ausschließlich innerhalb des Gehäuses angeordnet. Vorzugsweise ist die Schaltventilkammer, die Betätigungskammer und/oder die Rückstellkammer durch das Gehäuse gebildet. Das Gehäuse kann wenigstens zwei oder genau zwei Schalenteile, insbesondere Halbschalenteile, umfassen, zwischen denen das Schaltventilglied und gegebenenfalls das Trennglied gehalten sind. Das Gehäuse kann ein erstes, oberes Schalenteil aufweisen, das die Rückstellkammer, den Staudruckkanal und den Rückstelldruckkanal bilden.

Die Luftführung vom Rückstelldruckkanal, insbesondere der Rückstelldrossel, zum Staudruckkanal, insbesondere der Auslassdrossel, verläuft in einem Bogen am Umfang der Rückstellkammer. Vorzugsweise bildet das zweite, untere Schalenteil die Betätigungskammer des Aktors und den Stelldruckkanal. Der Entlüftungskanal ist in einem der beiden Schalenteile angeordnet, beispielsweise dem ersten Schalenteil, und der Luftversorgungskanal in dem anderen Schalenteil, beispielsweise dem zweiten Schalenteil, oder umgekehrt. Der Hauptluftkanal kann in dem ersten oder in dem zweiten Schalenteil gebildet sein. Vorzugsweise ist der Hauptluftkanal in dem zweiten Schalenteil gebildet. Die Schaltventilkammer ist vorzugsweise durch ein in der Trenn- oder Schnittebene des Gehäuses gemeinsam durch einen von dem ersten und dem zweiten Schalenteil umgebenen Hohlraum bereitgestellt. Die Schaltventilkammer kann einen zumindest teilweise umfänglichen, vorzugsweise vollumfänglichen, Ringkanal umfassen. In dem ersten Schalenteil ist vorzugsweise der Rückstelldruckkanal gebildet, der sich von der Schaltventilkammer zu der Rückstellkammer erstrecken kann. Das Trennglied, insbesondere die Membran, zum pneumatischen Trennen der Rückstellkammer von der Betätigungskammer ist vorzugsweise in der Trennebene zwischen dem ersten Schalenteil und dem zweiten Schalenteil gehalten. Das Schaltventilglied ist vorzugsweise in der Trennebene zwischen dem ersten Schalenteil und dem zweiten Schalenteil gehalten.

Gemäß einer Weiterbildung der Erfindung umfasst die Schaltventilkammer einen das Schaltventilglied zumindest teilumfänglich, insbesondere vollumfänglich, umgebenden Ringkanal. Der Ringkanal steht vorzugsweise kontinuierlich in fluidischer Kommunikation mit dem Hauptluftkanal. Der Ringkanal kann mit dem Luftversorgungskanal und/oder dem Entlüftungskanal in fluidischer Kommunikation stehen. Insbesondere verbindet der Rückstelldruckkanal die Rückstellkammer mit dem Ringkanal.

Gemäß einer anderen Weiterbildung der Erfindung, die mit den vorherigen kombinierbar ist, münden an derselben außenseitigen Anschlussfläche des Gehäuses sowohl ein Stelldruckkanal zum Bereitstellen von Druckluft für den ersten Steuerdruck in der Betätigungskammer, der Staudruckkanal, der Hauptluftkanal, der Entlüftungskanal als auch der Luftversorgungskanal. Soweit diese Ausführungen in Kombination mit der zuvor genannten Ausführung gebildet ist, wonach die des pneumatisch betätigte Schaltventil eine Steuerdrossel variabler Drosselweite aufweist, kann zusätzlich auch ein Manipulator für das bewegliche Drosselglied, beispielsweise eine Schraubendreher-Aufnahme einer Madenschraube, an derselben außenseitigen Anschlussfläche des Gehäuses angeordnet sein. Die Anordnung der Kanalmündungen und gegebenenfalls des Drosselglied-Manipulators an derselben außenseitigen Anschlussfläche des Gehäuses erlaubt eine besonders kompakte Bauweise und einfache Zugänglichkeit zu den Mündungen und dem Manipulator, insbesondere für Anordnungen, die pneumatische Komponenten, wie Ventile, umfassen. Es sei klar, dass das Gehäuse des pneumatisch betätigten Schaltventils verschiedene Formen haben kann. Eine bevorzugte Form eines Schaltventilgehäuses kann eine Quaderform sein. Ein Schaltventil mit quaderförmigem Gehäuse weist im Allgemeinen sechs außenseitige Flächen auf. Es kann bevorzugt sein, dass mit Ausnahme der Anschlussfläche die übrigen insbesondere fünf außenseitigen Flächen des Gehäuses frei von Mündungen pneumatischer Kanäle sind und/oder frei sind von Manipulatoren zum Einstellen eines beweglichen Drosselgliedes, der Rückstellfeder oder dergleichen.

Die Erfindung betrifft auch eine Ventilanordnung, die zwei pneumatisch betätigte Schaltventile umfasst, wobei wenigstens eines der zwei pneumatisch betätigten Schaltventile wie oben dargelegt ausgeführt ist. Gemäß einer besonderen Ausführung einer Ventilanordnung, die zwei pneumatisch betätigte Schaltventile umfasst, ist der Hauptluftkanal des ersten Schaltventils (erster Hauptluftkanal) mit einem Stelldruckkanal des zweiten Schaltventils (zweiter Stelldruckkanal) zum Bereitstellen von Druckluft an die Betätigungskammer des zweiten Schaltventils (zweite Betätigungskammer) verbunden. Es sei klar, dass ein "erster Kanal" im Allgemeinen auf einen Kanal des ersten Schaltventils und ein "zweiter Kanal" im Allgemeinen auf einen Kanal des zweiten Schaltventils verbindet.

Insbesondere ist der erste Hauptluftkanal über einen Selbstbetätigungskanal mit einem ersten Stelldruckkanal des ersten Schaltventils zum Bereitstellen von Druckluft an die Betätigungskammer des ersten Schaltventils angebunden. Gemäß einer Ausführung sind an den ersten Hauptluftkanal sowohl der Stelldruckkanal des ersten Schaltventils (erster Stelldruckkanal) als auch der zweite Stelldruckkanal angebunden. Die Verbindung von dem ersten Hauptluftkanal zu dem ersten Stelldruckkanal wird als Selbstbetätigungskanal bezeichnet. Zwischen dem Hauptluftkanal und den Stelldruckkanälen ist eine Kanalgabelung vorgesehen, wobei ausgehend vom ersten Hauptluftkanal pneumatische Druckluft einerseits an der Gabelung abgezweigt wird zu dem ersten Stelldruckkanal und andererseits an der Gabelung abgezweigt wird zu dem zweiten Stelldruckkanal. Die stromabwärts der Gabelung hinter dem Hauptluftkanal sich in Richtung des ersten Stelldruckkanals erstreckende Leitung wird als Selbstbetätigungskanal bezeichnet.

Gemäß einer Weiterbildung der Ventilanordnung weist der Selbstbetätigungskanal ein pneumatisches Verzögerungsglied auf. Ein pneumatisches Verzögerungsglied im Selbstbetätigungskanal bewirkt, dass ein an dem ersten Hauptluftkanal bereitgestelltes Druckluftsignal erst nach einem durch das Verzögerungsglied bewirkten zeitlichen Versatz den Stelldruckkanal erreicht. Beispielsweise kann mit dem pneumatischen Verzögerungsglied im Selbstbetätigungskanal bewirkt werden, dass eine Erhöhung des pneumatischen Luftdrucks im ersten Hauptluftkanal erst nach einem zeitlichen Versatz sich in einer Erhöhung des Druckes an dem ersten Stelldruckkanal bzw. dem ersten der ersten Betätigungskammer auswirkt. Das pneumatische Verzögerungsglied kann als ein nichtlineares Verzögerungsglied realisiert sein, dessen zeitliche Verzögerungswirkung abhängig ist von der Druckdifferenz zwischen erstem Hauptluftkanal und erstem Stelldruckkanal und/oder von dem Volumenstrom ist.

Das pneumatische Verzögerungsglied umfasst vorzugsweise einen Volumenspeicher und/oder eine Selbstbetätigungsdrossel. Die Selbstbetätigungsdrossel kann eine Drossel mit konstanter Drosselwirkung oder eine Drossel mit variabler Drosselung sein, die ein ortsfestes des Betätigung-Drosselglied und ein bewegliches Selbstbetätigungskanal-Drosselglied aufweist.

Die Ventilanordnung mit einem ersten pneumatisch betätigten Schaltventil, dessen erster Hauptluftkanal mit dessen erstem Stelldruckkanal verbunden ist, kann eine periodische, insbesondere zyklische, Eigenbetätigung bewirken, wobei die Zykluslänge mit dem Verzögerungsglied, insbesondere in Abhängigkeit von dem Verzögerungsglied zugeführten pneumatischen Arbeitsdruck und Volumenstrom, einstellbar ist.

Gemäß einer bevorzugten Ausführung ist das erste Schaltventil als erfindungsgemäßes pneumatisch betätigtes Schaltventil ausgeführt. Insbesondere kann das erste Schaltventil ausgeführt sein gemäß der oben beschriebenen bevorzugten Ausführungen, wobei der erste Rückstelldruckkanal die erste Schaltventilkammer mit der ersten Rückstellkammer verbindet und/oder wobei der Rückstelldruckkanal einer Steuerdrossel variabler Drosselweite aufweist. Gemäß einer bevorzugten Ausführung weist die in dem ersten Rückstelldruckkanal angeordnete Steuerdrossel eine konstante Drosselweite auf. Das zweite pneumatisch betätigte Schaltventil kann als konventionelles pneumatisch betätigtes Schaltventil ausgeführt sein. Es kann bevorzugt sein, dass das zweite Schaltventil als erfindungsgemäßes pneumatisch betätigtes Schaltventil ausgeführt ist. Insbesondere ist das zweite pneumatisch betätigte Schaltventil ausgeführt gemäß einer der wenigstens einer der oben beschriebenen bevorzugten Ausführungsformen, wobei der zweite Rückstelldruckkanal die zweite Schaltventilkammer mit der zweiten Rückstellkammer verbindet und/oder wobei die zweite Steuerdrossel im zweiten Rückstelldruckkanal eine konstanter Drosselweite hat.

Die Ventilanordnung kann insbesondere frei sein von elektrischen Antrieben, wie einem elektrischen Stellantrieb, einer Pumpe oder dergleichen, und/oder frei sein von elektrischen Aktoren, wie einem Solenoidventil. Die Ventilanordnung ist vorzugsweise frei von elektronischen Komponenten. Gemäß einer bevorzugten Ausführung besteht die Ventilanordnung aus pneumatischen und mechanischen Komponenten.

Die Erfindung betrifft auch ein Beatmungssystem, dass wenigstens ein erfindungsgemäßes pneumatisch betätigtes Schaltventil aufweist. Die Erfindung betrifft auch ein Beatmungssystem, dass eine erfindungsgemäße Ventilanordnung umfasst. Ein Beatmungssystem und insbesondere ein Beatmungssystem zur therapeutischen Verabreichung von mit Sauerstoff angereicherter Luft, haben in der Regel ein erhöhtes Brandrisiko zur Folge. Dabei kann es besonders vorteilhaft sein, derartige Systeme mit einem oder mehreren pneumatisch betätigten Schaltventilen auszustatten, um ein Entzündungsrisiko zu vermeiden, welches mit der Stromversorgung elektrisch betätigter Ventile einhergehen würde. Für lebenserhaltende therapeutische Systeme, wie Beatmungssysteme, ist die besonders hohe Verlässlichkeit und Ausfallsicherheit pneumatisch betätigter Schaltventile von besonderem Vorteil.

Bei einer Weiterbildung des erfindungsgemäßen Beatmungssystems ist zumindest die insbesondere zweite Schaltventilkammer sowie gegebenenfalls die insbesondere zweite Rückstellkammer und/oder die insbesondere zweite Steuerkammer mit einem Pneumatikfluid gefüllt, das zu wenigstens 30 %, insbesondere wenigstens 60 %, vorzugsweise wenigstens 90 % Sauerstoff aufweist.

Gemäß einer alternativen erfindungsgemäßen Weiterbildung, die mit der vorherigen kombinierbar ist, ist der Hauptluftkanal des pneumatisch betätigten Ventils, insbesondere der zweite Hauptluftkanal des zweiten pneumatisch betätigten Ventils der Ventilanordnungen mit einem pneumatischen Aktuator zur Balgbeaufschlagung verbunden, wobei das pneumatisch betätigte Schaltventil, insbesondere die Ventilanordnung, dazu eingerichtet ist, den pneumatischen Aktuator zyklisch mit Druckluft zu versorgen, um der Balgbeaufschlagung einen Arbeitsdruck bereitzustellen.

Weitere Aspekte der Erfindung sind in den Unteransprüchen angegeben. Weitere Vorteile, Merkmale und Aspekte der Erfindung sind durch die folgende Beschreibung bevorzugter Ausführungen der Erfindung anhand der beiliegenden Zeichnungen erläutert, in denen zeigen:
Figur 1a eine schematische Darstellung einer ersten Ausführung eines pneumatisch betätigten Schaltventils in einer zweiten Schließstellung des Schaltventilglieds;
Figur 1b eine schematische Darstellung des pneumatisch betätigten Schaltventils gemäß Figur la in der ersten Schließstellung des Schaltventilgliedes;
Figur 2 eine zweite Ausführung eines pneumatisch betätigten Schaltventils mit einer Steuerdrossel variabler Drosselweite;
Figur 3 eine perspektivische Ansicht einer weiteren Ausführung eines erfindungsgemäßen pneumatisch betätigten Schaltventils;
Figur 3a eine Schnittdarstellung des Schaltventils gemäß Figur 3 durch die Gehäuse Halbschalen, in der Auslassdrossel, Steuerdrossel und Rückstellkammer angeordnet sind;
Figur 3b eine Schnittansicht entlang der Schnittlinie B-B gemäß Figur 3;
Figur 3c eine Schnittansicht des Schaltventils Schnittlinie 10-C gemäß Figur 3;
Figur 3d eine Schnittansicht durch das Schaltventil gemäß Figur 3 E durch die Schnittlinie die-die;
Figur 3e eine Draufsicht auf das Schaltventils gemäß Figur 3;
Figur 4 eine schematische Darstellung einer erfindungsgemäßen Ventilanordnung;
Figur 5a ein Schaltdruck Diagramm, welche den Einatmenvorgang darstellt; und
Figur 5b ein Druckverlaufsdiagramm eines ausatmen Vorgangs.

Zur einfacheren Lesbarkeit und Verständlichkeit der Anmeldung werden im Folgenden für dieselben oder ähnliche Komponenten der dieselben oder ähnliche Bezugszeichen verwendet.

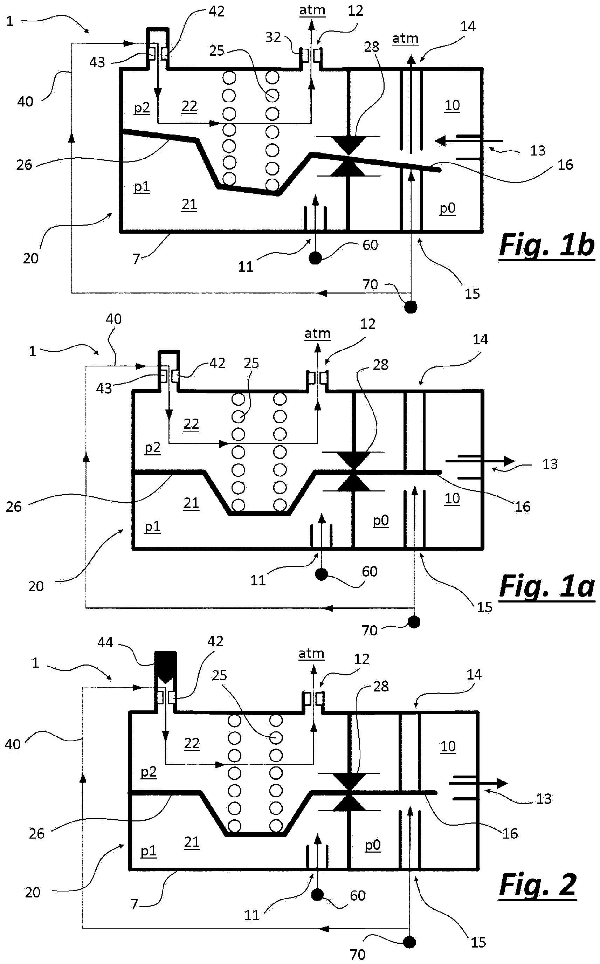

Die Figuren 1a und 1b zeigen eine schematische Darstellung einer ersten Ausführung eines erfindungsgemäßen pneumatisch betätigten Schaltventils 1.

Wie ein gattungsgemäßes pneumatisch betätigtes Schaltventil weist das erfindungsgemäße pneumatische betätigte Schaltventil 1 einen pneumatischen Aktor 20 auf, um einen Schaltventilglied 16 zu betätigen. Das Schaltventilglied 16 ist in der Schaltventilkammer 10 beweglich angeordnet. In die Schaltventilkammer 10 mündet ein Hauptluftkanal 13, ein Entlüftungskanal, der zu einer Drucksenke, wie der Atmosphäre atm führt, und ein Luftversorgungskanal 15, durch den Arbeit-Druckluft in die Schaltventilkammer 10 von einer Pneumatik-Quelle gelangt. Das Schaltventilglied 16 ist zwischen der in Figur 1a dargestellten ersten Schließstellung, In der das Schaltventilglied 16 den Entlüftungskanal 14 verschließt beweglichen eine in Figur 1 B dargestellte zweite Schließstellung, in der das Schaltventilglied 16 den Luftversorgungskanal 15 verschließt. In der ersten Schließstellung des Schaltventilglieds 16 kann die Druckluft von der Pneumatik-Quelle 70 durch den Luftversorgungskanal 15 in die Schaltventilkammer 10 und von der Schaltventilkammer 10 zu dem Hauptluftkanal 13 fließen. An den Hauptluftkanal 13 kann eine pneumatische Komponente, wie ein pneumatischer Aktor, angeschlossen sein. In der zweiten Schließstellung kann Pneumatikfluid von einer pneumatischen Komponente durch den Hauptluftkanal 13 in die Schaltventilkammer 10 und von dort aus durch den Entlüftungskanal 14 zu einer Druckluftsenke strömen. Der in der Schaltventilkammer 10 vorliegende Pneumatikdruck wird im Folgenden als Arbeitsdruck po bezeichnet. Der Arbeitsdruck po ist abhängig von der Stellung des Schaltventilgliedes 16 und kann im Bereich zwischen dem Druckniveau der Drucksenke, wie der Atmosphäre atm, und dem Druckniveau des Versorgungsdrucks pQ der pneumatischen Quelle 70 liegen.

Das Schaltventilglied 16 wird betätigt durch den pneumatischen Aktor 20. Der pneumatische Aktor 20 besteht aus einer Betätigungskammer 21 und eine Rückstellkammer 22. Die Betätigungskammer 21 und die Rückstellkammer 22 sind durch ein Trennglied 26 pneumatische voneinander separiert. Das Trennglied 26 kann als Balg oder gemäß der abgebildeten bevorzugten Ausführung als Membran 26 gebildet sein. Das Trennglied 26 ist beweglich und verbunden mit dem Schaltventilglied 16, sodass eine Bewegung des Trennglied des 26 eine Bewegung des schalt die des 16 veranlasst. Durch eine Verlagerung der Position des Trennglied des 26 kann das Schaltventilglied 16 zwischen seiner ersten Schließstellung und seiner zweiten Schließstellung hin- und her-bewegt werden. Die Verlagerung des Ventilgliedes 26 wird veranlasst durch die auf das Trennglied 26 wirkenden Kräfte. Ausgehend von der Steuerkammer 21 wirkt abhängig von einem ersten Steuerdruck p1 eine Druckkraft auf das Trennglied 26.

In der Rückstellkammer 22 ist eine Rückstellfeder 25 angeordnet, welche auf das Trennglied 26 drückt. Die Kraft der Rückstellfeder 25 wirkt entgegen der durch den ersten Steuerdruck p1 ausgeübten Druckkraft. In der Rückstellkammer 22 herrscht ein zweiter Steuerdruck p2 vor. Der zweite Steuerdruck p2 bewirkt eine auf das Trennglied 26 wirkende zweite Steuerdruckkraft, welche entgegen der durch den ersten Steuerdruck p1 ausgeübten Steuerdruckkraft wirkt. Bei konventionellen pneumatisch betätigten Schaltventilen entspricht die der zweite Steuerdruck p2 üblicherweise stets dem Druck einer Drucksenke, insbesondere der Atmosphäre atm. In dem pneumatischen Aktor 20 wirkt die erste Druckkraft verursachte durch den auf das Trennglied 26 wirkenden ersten Steuerdruck p1 entgegen dem durch den zweiten Steuerdruck p2 auf das Trennglied 26 wirkenden zweiten Druckkraft und die durch die Rückstellfeder 25 verursachte Rückstellkraft. Bei den pneumatisch betätigten Schaltventilen 1 gemäß der Figuren 1a und 2 bewirkt die Rückstellfeder 25 eine Vorspannung, die bei druckloser Betätigungs- und Rückstellkammer 21, 22 das Schaltventilglied 16 zum Einnehmen der ersten Schließstellung veranlasst. Das pneumatische Trennglied 26 ist über ein Gelenk 28, beispielsweise ein Festkörpergelenk, mit dem Schaltventilglied 16 verbunden.

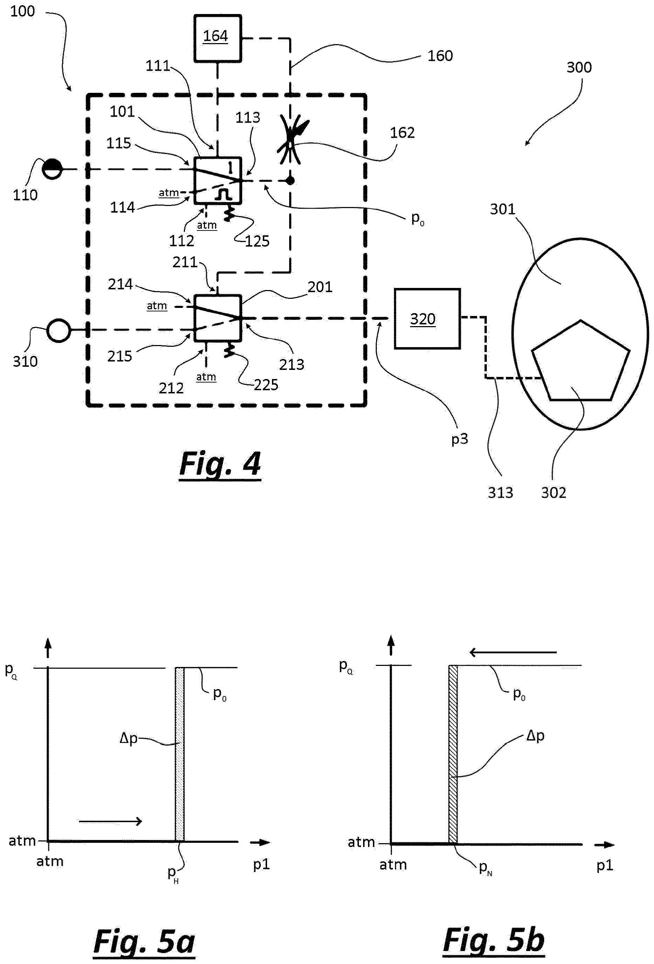

In einer anderen, nicht im Detail dargestellten Ausführung eines pneumatisch betätigten Schaltventils (etwa dem in Figur 4 dargestellten zweiten Schaltventil 201), kann die Rückstellfeder eine Vorspannung bewirken, die bei druckloser Betätigungs- und Rückstellkammer 21, 22 das Schaltventil 16 das Schaltventilglied 16 zum Einnehmen der zweiten Schließstellung veranlasst. Ein derart ausgeführtes pneumatisch betätigtes Schaltventil könnte beispielsweise durch Vertauschen von Entlüftungskanal 14 und Luftversorgungskanal 15 bei im Übrigen gleichbleibendem Aufbau des Schaltventils realisiert werden.

Der erste Steuerdruck p1 wird bereitgestellt durch ein durch den Druck von einer Pneumatikfluid-Quelle 60, die über den Stelldruckkanal 11 mit der Betätigungskammer 21 in fluidischer Verbindung steht. Die Pneumatik-Quelle 60 ist im Allgemeinen dazu eingerichtet, einen ersten Steuerdruck p1 für die Betätigungskammer 21 bereitzustellen, der wenigstens so groß ist wie der Druck einer Druckluftsenke, insbesondere der Atmosphäre, jedoch deutlich höher sein kann.

Wie unten in Bezug auf die erfindungsgemäße Ventilanordnungen 100 in Figur 4 dargelegt, kann beispielsweise der Stelldruckkanal 11 gespeist werden mit der Druckluft, die von einer Pneumatik-Quelle 70 durch den Hauptluftkanal 13 fließt. Bezugnehmend auf das zweite pneumatisch betätigte Schaltventil 201 in Figur 4 sei bemerkt, dass es auch denkbar ist, dass als Pneumatik-Quelle 60 einer anderen Komponente einer Ventilanordnung, beispielsweise ein anderes pneumatisch betätigtes Schaltventil 101 wirkt. Durch die Schaltventilkammer 10 kann eine pneumatische Komponente, die in fluidischer Verbindung mit dem Hauptluftkanal 13 steht, abhängig von der Stellung des Schaltventilglieds 16 wahlweise über den Luftversorgungskanal 15 mit Druckluft versorgt werden oder über den Entlüftungskanal 14 entlüftet werden.

Die Rückstellkammer 22 steht über einen Kanal in Verbindung mit der Druckluftsenke, wie der Atmosphäre. Bei der erfindungsgemäßen Ausführung eines pneumatisch betätigten Schaltventils 1 ist der Kanal, welcher die Rückstellkammer 22 mit der Drucksenke, insbesondere der Atmosphäre atm, verbindet, als Staudruckkanal 12 ausgeführt, der einer Auslassdrossel 32 aufweist. Die Auslassdrossel 32 bewirkt, dass ein der Rückstellkammer 22 bereitgestellter zweiter Steuerdruck p2, welcher größer ist als der Atmosphärendruck patm durch die Auslassdrossel 32 vom sofortigen entweichen abgehalten wird. Das entweichen des Steuerdrucks p2 aus der Rückstellkammer 22 wird durch die Auslassdrossel 32 gebremst.

Gemäß einer Ausführung eines erfindungsgemäßen pneumatisch betätigten Schaltventils 1 kann der zweite Steuerdruck p2 an die Rückstellkammer 22 bereitgestellt werden durch einen Rückstelldruckkanal 40, der aus der Pneumatik-Quelle 70 über den Luftversorgungskanal 15 versorgt wird. In dem Rückstelldruckkanal 40 kann eine Steuerdrossel 42 angeordnet sein, um zu bewirken, dass der Steuerdruck p2 einen bestimmten Druckwert geringer als den von der Druck-Quelle 70 bereitgestellten Versorgungsdruck pQ nicht überschreitet. Bei der in den Figuren 1a und 1b dargestellten Ausführung, bei der der Rückstelldruckkanal 40 stromaufwärts der Mündung des Luftversorgungskanals 15 in die Schaltventilkammer 10 von dem Luftversorgung Kanal 15 abgezweigt ist, kann der zweite Steuerdruck p2 einen Druckwert zwischen dem Druck, insbesondere Atmosphärendruck patm, der Druckluftsenke, insbesondere der Atmosphäre, und dem an der Pneumatikfluid-Quelle 70 bereitgestellten Versorgungsdruck pQ einnehmen. Der zweite Steuerdruck p2 ist abhängig von der Differenz zwischen dem Versorgungsdruck pQ und dem Druck der Drucksenke, insbesondere patm, und der Dimensionierung der Auslassdrossel 32 einerseits und der Steuerdrossel 42 andererseits. Durch geeignete Dimensionierung des Versorgungsdruckes pQ und der Drosselweite der Auslassdrossel 32 und der Steuerdrossel 42 kann der zweite Steuerdruck p2 vorbestimmt eingestellt werden, welcher die zweite Steuerdruckkraft zu Beaufschlagung das Trennglieds 26 entgegen der durch den ersten Steuerdruck p1 bereitgestellten ersten Steuerdruckkraft bewirkt.

Die Einstellung des zweiten Steuerdrucks p2 definiert den Schaltpunkt, ab welchem die erste Steuerdruckkraft die Rückstellkraft durch die zweite Steuerdruckkraft und die Federrückstellung der Rückstellfeder überwindet. Wenn der zweite Steuerdruck p2 gleich dem Druck der Drucksenke oder geringfügig größer als der Druck Drucksenke eingestellt ist, hat das pneumatisch betätigte Schaltventil 1 einen niedrigen Schaltpunkt pN, sodass die erste Steuerdruckkraft durch den ersten pneumatischen Steuerdruck p1 rasch die Rückstellkraft überwindet. Ist hingegen der zweite Steuerdruck p2 signifikant höher eingestellt als der Druck der Drucksenke, beispielsweise nahe dem Versorgungsdruck pQ, kann ein Schaltpunkt erreicht werden, der verhältnismäßig hoch ist. Dann muss der erste Steuerdruck p1 einen relativ hohen Schaltdruck pH überschreiten, um den das Schaltventilglied 16 zwischen der ersten Schließstellung und der zweiten zu bewegen. Eine Umschaltung bei einem niedrigen Schaltdruck pN ist in Figur 5b exemplarisch dargestellt. Eine Umschaltung bei einem hohen Schaltdruck pH ist exemplarisch in Figur 5a abgebildet. Der niedrige Schaltdruck pN liegt vorzugsweis in einem Bereich von 0,1 bar bis 1 bar, insbesondere bei 0,55 ±0,10 bar. Der hohe Schaltdruck pH liegt vorzugsweis in einem Bereich von 1,1 bar bis 2 bar, insbesondere bei 1,5 ±0,10 bar.

Figur 2 zeigt ein pneumatisch betätigtes Schaltventil 1, das sich im Wesentlichen nur durch die Art des der Steuerdrossel 42 im Rückstelldruckkanal 40 von dem zuvor bezügliche Figuren 1a und 1b beschriebenen pneumatisch betätigten Schaltventil 1 unterscheidet. In den Figuren 1a und 1b kann angenommen werden, dass die Steuerdrossel 42 und die Auslassdrossel 32 je eine konstante statische Drosselweite aufweisen.

Hiervon unterscheidet sich die Ausführung gemäß der Figur 2 dadurch, dass die Steuerdrossel 42 ein bewegliches Drosselglied 44 und ein unbewegliches Drosselglied 43 umfasst, wobei die Relativstellung des beweglichen Drosselgliedes 44 relativ zu dem statischen Drosselglied 43 der Steuerdrossel 42 variabel ist, um die Drosselweite einzustellen.

Durch Verwendung eines Drosselventils variabler Stellweite, das ein ortsfestes Drosselglied 43 und ein bewegliches Drosselglied 44 umfasst, kann der Durchströmungsquerschnitt der des Rückstelldruckkanals 40 variabel eingestellt werden, um die Einstellung des zweiten Steuerdrucks p2 in der Rückstellkammer 22 variabel niedriger als den bereitgestellten Versorgungsdruck pQ der Pneumatik-Quelle 70 einzustellen. Wird eine große Drosselweite eingestellt, kann der zweite Steuerdruck p2 an den Versorgungsdruck pQ angenähert werden. Wird die Drosselweite zwischen dem beweglichen Drosselglied 44 und dem ortsfesten Drosselglied 43 gering eingestellt, kann der zweite Steuerdruck p2 an den Druck der Drucksenke, insbesondere den Atmosphärendruck patm, angenähert werden. Gegebenenfalls kann der Rückstelldruckkanal 40 verschlossen werden, indem das bewegliche Drosselglied 44 in einen Berührkontakt mit dem ortsfesten Teil 43 der Steuerdrossel 42 gebracht wird, sodass der zweite Steuerdruck p2 gleich dem Druck der Drucksenke wird.

Alternativ oder zusätzlich kann es denkbar sein, die Auslassdrossel 32 mit einem statischen und einem beweglichen Drosselglied auszustatten, um den Druckunterschied zwischen der Drucksenke und der Rückstellkammer am Staudruckkanal variabel einstellbar zu gestalten (nicht näher dargestellt).

Durch die Verwendung eines beweglichen Drosselglieds 44 kann beispielsweise eine Serie von pneumatisch betätigten Schaltventilen günstig hergestellt werden, wobei eine Nachjustierung mithilfe des beweglichen Drosselglieds 44 eine Kalibrierung der pneumatisch betätigten Schaltventile 1 erlaubt, sodass diese eingerichtet werden können, um einen vorbestimmten Schaltdruck pN, pH mit verhältnismäßig schmalem Druck-Toleranzbereich Δp.

Es ist denkbar, dass für den betriebsgemäßen Gebrauch das bewegliche Drosselglied 44 in einer bestimmten Stellposition beispielsweise mit einem Siegel fixiert, reversibel fixiert, beispielsweise verklebt, oder irreversibel fixiert, beispielsweise verschweißt, sein kann. Alternativ oder zusätzlich ist es denkbar, dass im betriebsgemäßen Gebrauch das bewegliche Drosselglied mittels eines Manipulator 46, wie einer Imbusschlüssel-Aufnahme, einstellbar ist. Es sei klar, dass im betriebsgemäßen Zustand die Position des beweglichen Drosselglieds 44 unabhängig von einem auf das bewegliche Drosselglied 44 wirkenden Versorgungsdruck, zweiten Stelldruck und/oder Atmosphärendruck ortsfest ist. Das bewegliche Drosselglied 44 kann beispielsweise durch eine Madenschraube realisiert sein. Das bewegliche Drosselglied 44 kann selbstdichtend oder mit einer zusätzlichen Dichtung ausgeführt sein, sodass entlang der Längserstreckung des eine Leckageströmung vermieden ist.

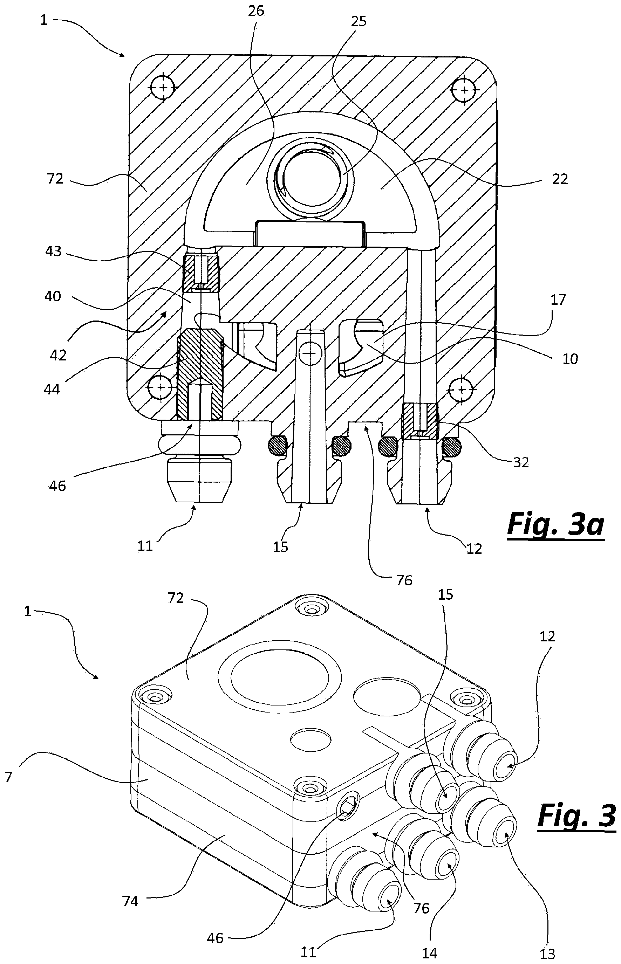

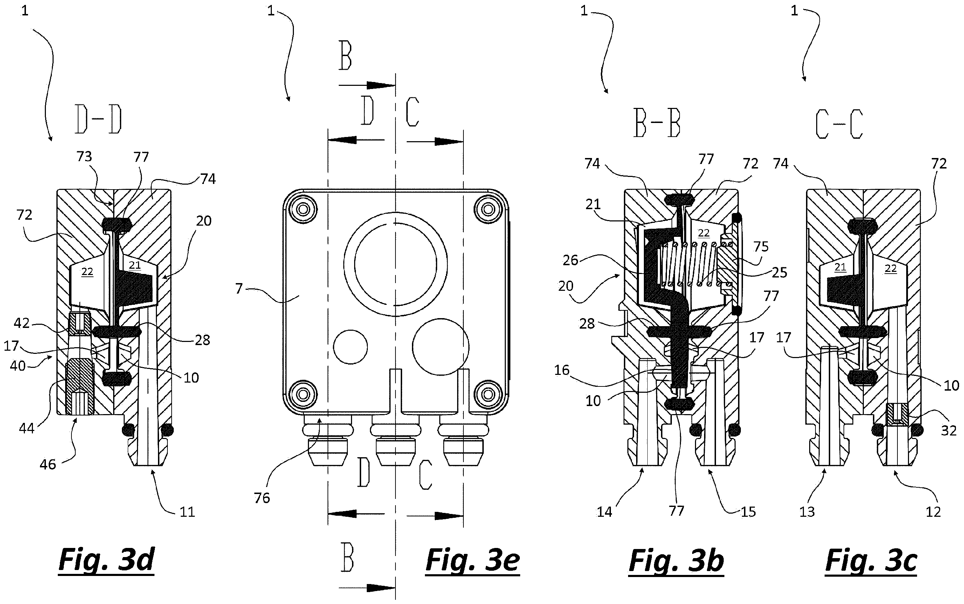

Figur 3 zeigt eine weitere Ausführung eines erfindungsgemäßen pneumatisch betätigten Schaltventils. Im Unterschied zu dem zuvor dargestellten pneumatisch betätigten Schaltventil 1 der Figur 1b zweigt der Rückstelldruckkanal 40 bei dem in den Figuren 3, 3a bis 3e gezeigten Schaltventils aus der Schaltventilkammer 10 ab, worauf im Folgenden näher eingegangen wird. Im Übrigen entspricht das Schaltventil gemäß der Figuren 3, 3a bis 3e im Wesentlichen dem in Figur 2 schematisch dargestellten Schaltventil 1.

Das pneumatisch betätigte Schaltventil 1 ist in einem Gehäuse 7 gebildet, dass es sich aus zwei Halbschalenteilen 72, 74 zusammensetzt. Eine Verschlusskappe 75 bildet teil eines Schalenteils, hält die Rückstellfeder 25 in Position bildet einen abgedichteten Verschluss der Rückstellkammer 22.

Das Gehäuse 7 ist quaderförmig ausgebildet und hat sechs Außenflächen. Von den sechs Außenflächen des Gehäuses 7 ist eine einzige als Anschlussfläche 76 gebildet, an der Anschlüsse für sämtliche Kanäle, d. h. den Stelldruckkanal 11, den Staudruckkanal 12, den Hauptluftkanal 13, den Entlüftungskanal 14 und den Luftversorgungskanal 15 gebildet sind. An der Anschlussfläche 76 ist auch der Manipulator 46 für das bewegliche Drosselglied 44 zugänglich. Diese Bauweise des pneumatisch betätigten Schaltventils 1 erlaubt eine besonders kompakte Bauweise. So können zahlreiche Pneumatikkomponenten, wie Ventile, insbesondere pneumatisch betätigte Schaltventile, mit ähnlichem oder gleichartigem quaderförmigen Gehäuse unmittelbar aneinander angrenzend angeordnet werden. Beispielsweise können Gehäuse 7 mehrerer pneumatischen Komponenten sandwichartig übereinander gestapelt werden, wobei sämtliche Anschlüsse zugänglich bleiben.

Der pneumatische Aufbau des Schaltventils 1 gemäß Figur 3, 3a - 3e entspricht im Wesentlichen dem pneumatischen Aufbau des beispielsweise bezüglich Figur 2 schematisch dargestellten Schaltventils 1. Die Schnittdarstellung Fig. 3b entlang der Schnittlinie B-B lässt die Schaltventilkammer 10 erkennen, in der das bewegliche Schaltventilglied 16 erkennen (hier in der den Entlüftungskanal 14 verschließenden ersten Schaltstellung). In die Schaltventilkammer 10 münden der Entlüftungskanal 14 und der Hauptluftkanal 13. Die Schaltventilkammer 10 umfasst einen Ringkanal 17, der das Schaltventilglied 16 vollumfänglich umgibt. Der Ringkanal 17 ist auch in den Schnittansichten Fig. 3c entlang der Schnittlinie C-C und Figur 3d entlang der Schnittlinie D-D zu erkennen. In den Ringkanal 17 und damit in die Schaltventilkammer mündet auch der Hauptluftkanal 13, der in Figur 3c ersichtlich ist.

Wie in Figur 3a zu sehen, verläuft die Luftführung vom Rückstelldruckkanal 40, insbesondere der Rückstelldrossel 42, zum Staudruckkanal 12, insbesondere der Auslassdrossel 32, in einem Bogen am Umfang der Rückstellkammer 22, die die Rückstellfeder 25 aufnimmt.

In Figur 3d ist ersichtlich, dass sich ein Rückstelldruckkanal 40 von dem der Ringkanal 17 zu der Rückstellkammer 22 erstreckt. In der Rückstelldruckkanal 40 sind ein ortsfestes Drosselglied 43 und ein bewegliches Drosselglied 44 angeordnet. Das bewegliche Drosselglied 44 kann die Öffnungsweite der Drossel 42 variabel einstellen, um den in der Rückstellkammer 22 herrschenden zweiten Steuerdruck p2 abhängig von dem in der Schaltventilkammer 10 herrschenden Arbeitsdruck po einzustellen.

Figur 3c zeigt auch den Staudruckkanal 12, von dem aus die Rückstellkammer 22 an eine Drucksenke, insbesondere die Atmosphäre atm entlüftet wird. In dem Staudruckkanal 12 ist eine Auslassdrossel 32 angeordnet. Der zweite Steuerdruck p2 in der Rückstellkammer 22 wird bei dieser Ausführung eingestellt durch die Dimensionierung der Auslassdrossel 32 und der Steuerdrossel 42.

Kooperierend mit der durch den zweiten Steuerdruck p2 bereitgestellten zweiten Steuerkraft wirkt die von der Feder 25 bereitgestellte Federrückstellung. Entgegen der zweiten Steuerkraft und der Rückstellkraft wirkt die erste Steuerkraft. Die erste Steuerkraft wird bereitgestellt durch den auf das Trennglied 16 wirkenden ersten Steuerdruck p1 in der Betätigungskammer 21. Zum empfangen des ersten Steuerdrucks p1 ist die Betätigungskammer 21 mit einem Stelldruckkanal 11 ausgestattet (Figur 3d).

Die Rückstellfeder 25 wird von einer Verschlusskappe 75 ortsfest an dem Gehäuse 7 gehalten. Die Verschlusskappe 75 verschließt die Rückstellkammer 22 luftdicht. Zu diesem Zweck kann die Verschlusskappe 75 mit einem Dichtmittel, wie einem O-Ring ausgestellt sein. Es ist denkbar, dass die Feder 25 einstellbar ist. Beispielsweise kann der Verschluss 75 dazu ausgestaltet sein, Feder 25 unterschiedlich tiefe in das Gehäuse 7 einzusetzen.

Abgesehen von der Anordnung des Rückstelldruckkanals 40 zwischen Schaltventilkammer 10 und Rückstellkammer 22 entspricht die Wirkweise des in den Figuren 3, 3a bis 3e dargestellten pneumatisch betätigten Schaltventils 1 der Wirkweise des zuvor bezüglich der Figuren 1a, 1b und 2 beschriebenen Schaltventil 1.

Die Anordnung des Rückstelldruckkanals 40 zum Verbinden der Rückstellkammer 22 und Schaltventilkammer 10 könnte alternativ realisiert sein durch eine Verbindung der Rückstellkammer 22 über einen nicht näher dargestellten Rückstelldruckkanal 40 mit dem Hauptluftkanal 13. Es sei angenommen, dass in dem Hauptluftkanal 13 und Schaltventilkammer 10 im Wesentlichen derselbe Druck, nämlich der Arbeitsdruck po, vorherrscht.

Indem der Arbeitsdruck po zur Versorgung der Rückstellkammer 22 mit Druckluft für den zweiten Steuerdruck p2 verwendet wird, kann auf besonders einfache Weise ein erfindungsgemäßes pneumatisch betätigtes Schaltventil 1 realisiert werden, welches für eine periodische, insbesondere zyklische Schaltung eingerichtet ist. Abhängig von der Schaltstellung des Schaltventilgliedes 10 stellt der Rückstelldruckkanal eine Verbindung der Rückstellkammer 22 entweder in der ersten Schließstellung durch den Luftversorgungskanal 15 zu der Druckluftquelle oder in der zweiten Schließstellung eine Verbindung zwischen dem der Rückstellkammer 22 über den Entlüftungskanal 14 mit einer Drucksenke, insbesondere der Atmosphäre. Abhängig von der Schließstellung des Schaltventils 1 kann also die Rückstellkraft eingestellt werden, indem der zweite Steuerdruck zu abhängig von der Schaltstellung verringert wird oder erhöht wird.

Wie in den Schnittdarstellungen gemäß Figur 3b, 3c oder 3d zu erkennen ist, setzt sich das Gehäuse 7 zusammen aus einer oberen Halbschale 72 und einer unteren Halbschale 74. Die Schalenteile 72, 74 liegen entlang einer Trennebene 73 aneinander. In der Trenn- oder Schnittebene 73 können vorzugsweise das Schaltventilglied 16 und/oder des Trendglied 26 sowie gegebenenfalls das Gelenk 28 gehalten sein.

Die erste Halbschale 72 bildet die Rückstellkammer 22, den Rückstelldruckkanal 40, den Staudruckkanal 12 und einen Luftkanal, beispielsweise den Luftversorgungskanal 15. Die zweite Gehäuse Halbschalen 74 bildet die Betätigungskammer 21, den Stelldruckkanal 11, den Hauptluftkanal 13 und einen weiteren Luftkanal, beispielsweise den Entlüftungskanal 14. Die Schaltventilkammer 10 wird gemeinsam durch die erste Gehäusehalbschale 72 und die zweite Gehäuse Halbschalen 74 gebildet. Die Schaltventilkammer 10 steht in fluidischer Verbindung mit einem Ringkanal 17, welcher sowohl in der ersten Halbschale 72 also auch in der zweiten Halbschale 74 gebildet ist.

In der Trennebene 73 zwischen der ersten Halbschale 72 und der zweiten Halbschale 74 ist das pneumatische Trennglied 26 zum Unterteilen des pneumatischen Aktors 20 in die Betätigungskammer 21 und die Rückstellkammer 22 angeordnet. In der Trennebene 73 ist auch das Schaltventilglied 16 angeordnet, welches verbunden mit dem Trennglied 26 gebildet sein kann. Die erste Halbschale 72 und die zweite Halbschalen 74 können beispielsweise mittels Schrauben (in Figur 3 angedeutet) gegeneinander verpresst werden, um einen abdichtenden Verschluss zu bilden. In der Trennebene 73 können Dichtmittel 77 angeordnet sein, um eine Leckage von Pneumatikfluid entlang der Trennebene 73 zu vermeiden. Das Dichtmittel 77 kann beispielsweise durch einen oder mehrere O-Ringe realisiert sein. Alternativ kann, wie dargestellt, dass Dichtmittel 77 einstückig mit dem Trennglied 26 und/oder dem Schaltventilglied 16 gebildet sein.

Bezugnehmend auf die in Figur 4 dargestellten Ventilanordnungen wird ein erstes pneumatisch betätigtes Schaltventil Bezugszeichen 101 und ein zweites pneumatisch betätigtes Schaltventil Bezugszeichen 201 bezeichnet. In verschiedenen erfindungsgemäßen Ausführungen einer Ventilanordnung kann das erste Schaltventil 101, und/oder das zweite Schaltventil 201 ein erfindungsgemäßes pneumatisch betätigtes Schaltventil sein. Für das erste pneumatische Schaltventil und das zweite pneumatische Schaltventil 101, 201 der Ventilanordnungen 100 werden im Vergleich zu den zuvor beschriebenen pneumatisch betätigten Schaltventilen um 100 bzw. 200 erhöhte Bezugszeichen verwendet. Es sei klar, dass bei verschiedenen erfindungsgemäßen Ausführungen einer Ventilanordnung keines der beiden Schaltventile oder nur das erste oder nur das zweite Schaltventil ein konventionelles pneumatisches Schaltventile sind. Alternativ können das erste pneumatische Schaltventil und/oder das zweite pneumatische Schaltventil verschiedenartige erfindungsgemäße pneumatische Schaltventile sein.

Figur 4 zeigt eine Ventilanordnung, die zwei pneumatisch betätigte Schaltventile aufweist. Gemäß einer bevorzugten Ausführung ist nur das erste Schaltventilglied 101 der Ventilanordnung 100 als erfindungsgemäßes pneumatisch betätigtes Schaltventilglied ausgeführt. Gemäß einer anderen Ausführung ist nur das zweite pneumatische Stellventil 20als erfindungsgemäßes pneumatisches Stellventil 1 wie oben beschrieben ausgebildet. Gemäß einer dritten alternativen Ausgestaltung können sowohl das erste pneumatische Stellventil als 1 als auch das zweite pneumatische Stellventil 201 als erfindungsgemäße pneumatische Stellventil gebildet sein.

Im Hinblick auf die folgende Beschreibung einer bevorzugten Ausführung der Erfindung wird eine exemplarische Ventilanordnung beschrieben, bei der das erste pneumatisch betätigte Schaltventil 101 der Ventilanordnung 100 mit einer Steuerdrossel 42 variabler Drosselweite realisiert ist, wie das oben beschriebe pneumatisch betätigte Schaltventil gemäß Figur 2 oder 3. Ferner wird exemplarisch angenommen, dass das zweite pneumatisch betätigte Schaltventil 201 mit einem Rückstelldruckkanal 40 gebildet ist, in dem eine statische Steuerdrossel 42 konstanter Drosselweite vorgesehen ist, wie bei der oben hinsichtlich der Figuren 1a und 1b beschriebenen Ausführung. Abweichend von der in Figur 1a und 1b dargestellten Ausführung eines pneumatisch betätigten Schaltventils, das in drucklosen Zustand des Aktors die erste Schließstellung einnimmt und Entlüftungskanal 14, 114 schließt, befindet sich das in Figur 4 skizzierte zweite Stellventil im drucklosen Zustand in der zweiten Schließstellung, in der der Luftversorgungskanal 215 geschlossen ist. Ferner sei angenommen, dass der Rückstelldruckkanal 40 der Schaltventile 101, 201 eine pneumatische Verbindung zwischen der jeweiligen Schaltventilkammer 10 und der jeweiligen Rückstellkammer 22 bereitstellt.

Die in Figur 4 dargestellte Ventilanordnung 100 ist Teil einer Beatmungsvorrichtung 300 zum therapeutischen Unterstützen oder Ersetzen der natürlichen Atemleistung eines Menschen 301. Der Mensch 301 ist mit einer Beatmungsmaske 302 ausgestattet. Die Maske 302 kann aus einem pneumatischen Aktuator 320 gespeist werden, beispielsweise einem Balgaktuator, der betätigt wird durch den im zweiten Hauptluftkanal 213 bereitgestellten pneumatischen Arbeitsdruck p3.

Von der Ventilanordnungen 100 wird einem zweiten Hauptluftkanal 213 ein zyklisch wechselnde Arbeitsdruck p3 bereitgestellt, um den pneumatischen Aktuator 320 zur Beatmung des Menschen 301 zu betätigen. An den zweiten Luftversorgungskanal 215 des zweiten Schaltventils 201 ist eine Beatmungsfluid-Quelle 310 angeschlossen, die im Allgemeinen auch als Pneumatikdruck-Quelle bezeichnet sein kann, angeschlossen. Abhängig von der Schaltstellung des pneumatisch betätigten Schaltventils 201 wird der zweite Hauptluftkanal 213 entweder zur Speisung des Arbeitsdrucks p3 von der Pneumatikdruck-Quelle 310 mit dem Luftversorgungskanal 215 verbunden. Oder der zweite Hauptluftkanal 213 wird in der anderen Schaltstellung zum Entlüften und Absenken des Arbeitsdrucks p3 verbunden mit der Entlüftungsleitung 214.

Das zweite pneumatisch betätigte Schaltventil 201 weist einen zweiten Stelldruckkanal 211 zur Verbindung mit der (zweiten) Betätigungskammer 21 (nicht näher dargestellt) des zweiten pneumatisch betätigten Schaltventils 201 auf. Im Stelldruckkanal 211 liegt der erste Steuerdruck p201 des zweiten Schaltventils 201 an. Wenn der erste Steuerdruck an dem zweiten Schaltventil 201 einen wahlweise niedrigen (oder hohen) ersten Schalt-Schwell-Druck pN (oder pH) überschreitet, wird das zweite pneumatisch betätigte Schaltventil 201 in die zweite Schließstellung verbracht. Wenn der erste Steuerdruck p1 des zweiten Schaltventils 201 unter einer vorbestimmten hohen (oder niedrigen) Schalt-Schwelldruckwerk pH bzw. pN absinkt, wird das zweite Schaltventil 201 in die zweite Schließstellung verbracht, sodass eine Entlüftung des Abluftkanals 213 erfolgt.

Der erste Steuerdruck p201 des zweiten Schaltventils 201 entspricht dem am ersten Hauptluftkanal 113 des ersten Schaltventils 101 bereitgestellten Arbeitsdruck po. In der ersten Schaltstellung des ersten pneumatisch betätigten Schaltventils 101 wird der erste Hauptluftkanal mit einem Arbeitsdruck po beaufschlagt, der durch den Versorgungsträger der Pneumatikfluid-Quelle 110 am ersten Luftversorgungskanal des ersten Schaltventils 101 bestimmt ist. In der zweiten Schaltstellung des ersten pneumatisch betätigten Schaltventils 101 wird der Arbeitsdruck po am ersten Hauptluftkanal 113 an eine Drucksenke, insbesondere Atmosphärendruck patm angeglichen, indem der erste Hauptluftkanal 113 mit dem ersten Entlüftungskanal 114 verbunden wird.

Der erste Steuerdruck p101 wird durch den am Hauptluftkanal 113 abgegebenen ersten Arbeitsdruck po mit einem pneumatischen Selbstbetätigungskanal 160 bereitgestellt, welcher den ersten Hauptluftkanal 113 mit dem ersten Stelldruckkanal 111 verbindet. Im ersten Selbstbetätigungskanal 160 ist ein pneumatisches Verzögerungsglied angeordnet, welches sich zusammensetzt aus einem Volumenspeicher 164 und eine Selbstbetätigungsdrossel 162.

Das pneumatische Verzögerungsglied im Selbstbetätigungskanal 160 bewirkt, dass der erste Steuerdruck p101 einen zeitlichen Verlauf erfährt, der gegenüber dem zeitlichen Verlauf des Arbeitsdrucks po am ersten Hauptluftkanal 113 zeitlich verschoben ist. Wenn das erste pneumatisch betätigte Schaltventil 1 in seiner ersten Schaltstellung die Luftversorgung 115 mit dem Hauptluftkanal 113 verbindet, steigt der Arbeitsdruck po im ersten Hauptluftkanal 113 rasch in etwa auf den Wert des Versorgungsdruckes der Pneumatikfluid-Quelle 110 an. Gegenüber dem Verlauf des Arbeitsdrucks po ist der Verlauf des ersten Steuerdrucks p101 des ersten, betätigten Schaltventils 101 verlangsamt, da die Selbstbetätigungsdrossel 162 einen unmittelbaren rapiden Druckanstieg des ersten Steuerdrucks p101 verhindern und weil der Volumenspeicher 164 ebenfalls eine verzögernde Wirkung auf die Änderung des ersten Steuerdrucks p101 des ersten pneumatisch betätigten Schaltventils 101 hat. Das Volumen des Druckspeichers 164 wird langsam über die Drossel 162 gefüllt und baut einen Schaltdruck auf.

Gleichsam entlüftet das Volumen im Druckspeicher 164 langsam, entsprechend des Durchlasses der insbesondere einstellbaren Drossel 162. Wenn umgekehrt in der zweiten Schaltstellung des ersten pneumatisch betätigten Schaltventils 101 der Hauptluftkanal 113 mit dem ersten Entlüftungskanal 114 verbunden ist, fällt der Arbeitsdruck po rapide auf das Niveau des Drucks der Drucksenke, insbesondere dem Atmosphärendruck patm ab. Wiederum verhindern die Selbstbetätigungsdrossel 162 und der Volumenspeicher 164 einen sofortigen Angleich des ersten Steuerdrucks p101 an den ersten Arbeitsdruck po. Der Volumenspeicher 164 stellt für eine gewisse Zeit weiterhin einen ersten Steuerdruck p101 bereit, welcher höher ist als der Arbeitsdruck po am ersten Hauptluftkanal 113. Mittels der Drossel 162 kann eine Zykluszeit eingestellt werden.

Die Pneumatikfluid-Quellen 110 und 310 können dieselbe Pneumatikfluid-Quelle sein oder aus derselben Pneumatikfluid-Quelle (nicht dargestellt) gespeist werden. Beispielsweise können die Pneumatikfluid-Quellen 110 und 310 aus ein und derselben Sauerstoffflasche gespeist werden, wobei der Sauerstoffanteil in dem Pneumatikfluid wenigstens 30 Vol.-%, insbesondere wenigstens 50 Vol.-%, vorzugsweise wenigstens 90% betragen kann. Der Versorgungsdruck pQ einer Pneumatikfluid-Quelle kann insbesondere zwischen 1,2 und 3,0 bar liegen, beispielsweise bei 1,5 bar ±0,25 bar. Im Allgemeinen bezeichnen die Pneumatikfluid-Quellen einzelne Quellen für Pneumatikfluid zur Anbindung an den ersten oder zweiten Luftversorgungskanals 115, 215 des ersten und/oder zweiten pneumatisch betätigten Schaltventils 101, 201.

Bei der oben beschriebenen Ventilanordnungen 100 wird an den ersten und zweiten Ventile 115, 215 eine Pneumatikdruck-Quelle 110 und/oder 310 angeschlossen. Wie die in den vorigen Figur 1 bis 3 dargestellten pneumatisch betätigten Schaltventile sind die in Figur 4 dargestellten betätigten Schaltventile 101, 201 mithilfe einer ersten Rückstellfeder 125 bzw. einer zweiten Rückstellfeder 225 vorgespannt. in einem Zustand, in dem die Steuerkammer 21 und die Rückstellkammer 22 nicht druckbeaufschlagt sind oder erster und zweiter Steuerdruck im Wesentlichen gleich sind, ist die Schaltstellung des pneumatisch betätigten Schaltventils durch die Rückstellfeder 125, 225 bestimmt.

In der ersten Schaltstellung ist der erste Entlüftungskanal 114 verschlossen. Bei dem zweiten pneumatisch betätigten Schaltventil 201 ist in der Ruhelage der Schaltventile durch die Rückstellwirkung der zweiten Rückstellfeder 225 die zweite Schaltstellung erwirkt. Das heißt, bei dem zweiten pneumatisch betätigten Schaltventil 201 gemäß Figur 4 ist ohne Druckbeaufschlagung der Steuerkammer 21 und Betätigungskammer 22 der Entlüftungskanal 214 mit dem zweiten Hauptluftkanal 213 verbunden.

In Folge der Bereitstellung von Druckluft mit dem Versorgungsdruck pQ von der ersten Pneumatikfluid-Quelle 110 wird allmählich der erste Steuerdruck p101 an der ersten Betätigungskammer 21 des ersten Schaltventils durch den Selbstbetätigungskanal 160 erhöht. Wenn der erste Steuerdruck p1 auf einen Wert oberhalb eines Schalt-Schwell-Druckwertes ansteigt, wird das erste pneumatisch betätigte Schaltventil 101 von seiner ersten Schließstellung in die zweite Schließstellung verfahren. Der Arbeitsdruck po wird dann an den beispielsweise Atmosphärendruck der Druckluftsenke am ersten Entlüftungskanal 114 angelegt. Über den Selbstbetätigungskanal fällt langsam der erste Steuerdruck p101.

In der ersten Schließstellung des ersten pneumatisch betätigten Schaltventils 101 ist die Rückstellkammer 22 über die Rückstelldruckkanal 40 mit der Schaltventilkammer 10 verbunden, in der ebenfalls der Arbeitsdruck herrscht. Dadurch muss der erste Steuerdruck 101 zunächst einen hohen Schaltdruck pH (Fig. 5a) überwinden, bevor der erste Steuerdruck 1 das pneumatisch betätigte Schaltventil 101 veranlasst, in seine zweite Schaltstellung zu wechseln (Fig. 5b).

In der zweiten schalt- bzw. Schließstellung herrscht in der Schaltventilkammer 10 der Druck der Druckluftsenke, insbesondere Atmosphärendruck patm vor. sodass in der Rückstellkammer 22 eine niedrige Rückstellkraft bereitgestellt ist. Dadurch muss der erste Steuerdruck p101 nicht bloß den hohen Schaltdruck pH unterschreiten, sondern einen signifikant niedrigeren niedrigen Schalt-Schwell-Druck pN, bevor das zweite pneumatisch betätigte Schaltventil 201 veranlasst wird, von der zweiten Schließstellung wieder in die erste Schließstellung zu wechseln (Fig. 5a).

Infolge der umgekehrten Vorspannung des zweiten Schaltventils 201 muss ein erster Steuerdruck p201 des zweiten Ventils 201 nur eine niedrige Schalt-Druck-Schwelle pN überschreiten, um in die erste Schaltstellung zu verfahren, in welcher der zweite Arbeitsdruck p2 maßgeblich durch den Versorgungsdruck pQ von der zweiten Pneumatikdruck-Quelle 13 bestimmt wird. Umgekehrt genügt es, wenn der am zweiten Schaltventil anliegende zweite Steuerdruck p2 bis unterhalb des hohen Druck-Schalt-Schwellenwertes pH abfällt, um das zweite pneumatisch betätigte Schaltventil 201 aus der ersten Schaltstellung in die zweite Schaltstellung zu verbringen.

Die in der vorstehenden Beschreibung, den Figuren und den Ansprüchen offenbarten Merkmale können sowohl einzeln als auch in beliebiger Kombination für die Realisierung der Erfindung in den verschiedenen Ausgestaltungen von Bedeutung sein.

### Bezugszeichenliste

- 1, 101, 201: pneumatisch betätigtes Schaltventil
- 7: Gehäuse
- 10: Schaltventilkammer
- 11, 111, 211: Stelldruckkanal
- 12, 112, 212: Staudruckkanal
- 13, 113, 213: Hauptluftkanal
- 14, 114, 214: Entlüftungskanal
- 15, 115, 215: Luftversorgungskanal
- 16: Schaltventilglied
- 17: Ringkanal
- 20: pneumatischer Aktor
- 21: Betätigungskammer
- 22: Rückstellkammer
- 25, 125, 225: Feder
- 26: Trennglied
- 28: Gelenk
- 32: Auslassdrossel
- 40: Rückstelldruckkanal
- 42: Steuerdrossel
- 43: statisches Drosselglied
- 44: bewegliches Drosselglied
- 60, 70, 110, 310: Pneumatikfluid-Quelle
- 72, 74: Schalenteile
- 73: Schnittebene
- 75: Verschluss
- 76: Anschlussfläche
- 77: Dichtmittel
- 100: Ventilanordnung
- 160: Selbstbetätigungskanal
- 162: Selbstbetätigungsdrossel
- 164: Volumenspeicher
- 300: Beatmungssystem
- 301: Mensch
- 302: Beatmungsmaske
- 313: Versorgungsleitung
- 320: pneumatischer Aktuator

- atm: Atmosphäre
- p₀: Arbeitsdruck
- p₁, P₁₀₁, p₂₀₁: erster Steuerdruck
- p₂: zweiter Steuerdruck
- p₃: Arbeitsdruck
- p_{H}: hoher Schalt-Schwelldruck
- p_{N}: niedriger Schalt-Schwelldruck
- p_{Q}: Versorgungsdruck
- Δp: Toleranzbreite

## Patentansprüche

1. Pneumatisch betätigtes Schaltventil (1, 101, 201)
mit einer Schaltventilkammer (10), in die drei Luftkanäle (13, 14, 15, 113, 114, 115, 213, 214, 215) münden, wobei die drei Luftkanäle (13, 14, 15, 113, 114, 115, 213, 214, 215) einen Luftversorgungskanal (15, 115, 215) zum Empfangen von Druckluft von einer Druckluftversorgungsquelle (70, 110, 310), einen Hauptluftkanal (13, 113, 213) zum Be- und Entlüften wenigstens einer anderen pneumatischen Komponente und einen Entlüftungskanal (14, 114, 214) zum Abgeben von Druckluft an eine Drucksenke, wie die Atmosphäre (atm), umfassen,
wobei in der Schaltventilkammer (10) ein Schaltventilglied (16) angeordnet ist, um den Luftversorgungskanal (15, 115, 215) oder den Entlüftungskanal (14, 114, 214) zu verschließen,
und mit einem pneumatischen Aktor (20) zum Betätigen des Schaltventilglieds (16), der mit einem als Membran oder Balg ausgestalteten Trennglied (26) pneumatisch unterteilt ist in eine Betätigungskammer (21) und eine Rückstellkammer (22), wobei ein erster Steuerdruck (p1) in der Betätigungskammer (21) entgegen einer Federrückstellung und entgegen einem zweiten Steuerdruck (p2) in der Rückstellkammer (22) wirkt,
**dadurch gekennzeichnet, dass**
die Rückstellkammer (22) einen Rückstelldruckkanal (40) zum Bereitstellen von Druckluft an die Rückstellkammer (22) und einen mit einer Auslassdrossel (32) ausgestatteten Staudruckkanal (12, 112, 212) zum Abgeben von Druckluft an eine Drucksenke, wie die Atmosphäre (atm), aufweist.

2. Pneumatisch betätigtes Schaltventil (1, 101, 201) nach Anspruch 1, **dadurch gekennzeichnet, dass** der Rückstelldruckkanal (40) verbunden ist mit dem Hauptluftkanal (13, 113, 213) und/oder dem Luftversorgungskanal (15, 115, 215) und/oder wobei der Rückstelldruckkanal (40) die Schaltventilkammer (10) mit der Rückstellkammer (22) verbindet.

3. Pneumatisch betätigtes Schaltventil (1, 101, 201) nach Anspruch 1 oder 2, **dadurch gekennzeichnet, dass** in dem Rückstelldruckkanal (40) eine Steuerdrossel (42) zum Einstellen des zweiten Steuerdrucks (p2) angeordnet ist.

4. Pneumatisch betätigtes Schaltventil (1, 101, 201) nach Anspruch 3, **dadurch gekennzeichnet, dass** die Steuerdrossel (42) eine variable Drosselweite aufweist, wobei insbesondere die Steuerdrossel (42) ein bewegliches Drosselglied (44), wie eine Madenschraube, umfasst.

5. Pneumatisch betätigtes Schaltventil (1, 101, 201) nach einem der vorstehenden Ansprüche, **dadurch gekennzeichnet, dass** die Federrückstellung einstellbar ist, wobei insbesondere eine Rückstellfeder (25, 125, 225) des pneumatischen Aktors (20) ein Stellmittel umfasst, um einen unteren Mindestschwellwert der Rückstellkraft einzustellen, welche wenigstens entgegen dem ersten Steuerdruck (p1) in der Betätigungskammer (21) wirkt.

6. Pneumatisch betätigtes Schaltventil (1, 101, 201) nach einem der vorstehenden Ansprüche, **dadurch gekennzeichnet, dass** der pneumatische Aktor (20) und die Schaltventilkammer (10) in demselben Gehäuse (7) untergebracht sind, wobei insbesondere das Gehäuse (7) wenigstens oder genau zwei Schalenteile (72, 74) umfasst, zwischen denen das Schaltventilglied (16) und/oder das Trennglied (26) gehalten ist.

7. Pneumatisch betätigtes Schaltventil (1, 101, 201) nach Anspruch 6, **dadurch gekennzeichnet, dass** die Schaltventilkammer (10) einen das Schaltventilglied (16) zumindest teilumfänglich, insbesondere vollumfänglich, umgebenden Ringkanal (17) umfasst, der in fluidischer Kommunikation mit dem Hauptluftkanal (13, 113, 213) und dem Luftversorgungskanal (15, 115, 215) und/oder dem Entlüftungskanal (14, 114, 214) steht, wobei insbesondere der Rückstelldruckkanal (40) die Rückstellkammer (22) mit dem Ringkanal (17) verbindet.

8. Pneumatisch betätigtes Schaltventil (1, 101, 201) nach einem der Ansprüche 6 oder 7, **dadurch gekennzeichnet, dass** an derselben außenseitigen Anschlussfläche (76) des Gehäuses (7) sowohl ein Stelldruckkanal (11, 111, 211) zum Bereitstellen von Druckluft an die Betätigungskammer (21), der Staudruckkanal (12, 112, 212), der Hauptluftkanal (13, 113, 213), der Entlüftungskanal (14, 114, 214) als auch der Luftversorgungskanal (15, 115, 215) münden, wobei insbesondere an der Anschlussfläche (76) ein Manipulator (46) für das bewegliche Drosselglied (44) angeordnet ist.

9. Ventilanordnung (100), umfassend zwei pneumatisch betätigte Schaltventile (1, 101, 201), wobei wenigstens eines der zwei pneumatisch betätigten Schaltventile (1, 101, 201) nach einem der Ansprüche 1 bis 8 ausgeführt ist, wobei der Hauptluftkanal (13, 113) des ersten Schaltventils (1, 101) mit einem Stelldruckkanal (11, 211) zum Bereitstellen von Druckluft an die Betätigungskammer (21) des zweiten Schaltventils (1, 201) verbunden ist,
**dadurch gekennzeichnet, dass**
der Hauptluftkanal (13, 113) des ersten Schaltventils (1, 101) über einen Selbstbetätigungskanal (160) mit einem Stelldruckkanal (11, 111) zum Bereitstellen von Druckluft an die Betätigungskammer (21) des ersten Schaltventils (1, 101) verbunden ist.

10. Ventilanordnung nach Anspruch 9, **dadurch gekennzeichnet, dass** der Selbstbetätigungskanal (160) ein pneumatisches Verzögerungsglied umfasst, wobei insbesondere das Verzögerungsglied einen Volumenspeicher (164) und/oder eine Selbstbetätigungsdrossel (162) umfasst.

11. Beatmungssystem (300) umfassend eine Ventilanordnung nach einem der Ansprüche 9 oder 10.

12. Beatmungssystem nach Anspruch 11 zur Balgbeatmung, **dadurch gekennzeichnet, dass** der Hauptluftkanal (13, 213) mit einem pneumatischen Aktuator (320) zur Balgbeaufschlagung verbunden ist, wobei die Ventilanordnung (100) dazu eingerichtet ist, den pneumatischen Aktuator (320) zyklisch mit Druckluft zu versorgen.

## Claims

1. Pneumatically actuated switching valve (1, 101, 201)
with a switching valve chamber (10), into which three air channels (13, 14, 15, 113, 114, 115, 213, 214, 215) open, wherein the three air channels (13, 14, 15, 113, 114, 115, 213, 214, 215) comprise an air supply channel (15, 115, 215) for receiving compressed air from a compressed air supply source (70, 110, 310), a main air channel (13, 113, 213) for venting at least one other pneumatic component and a venting channel (14, 114, 214) for discharging compressed air to a pressure sink, such as the atmosphere (atm),
wherein in the switching valve chamber (10) is arranged a switching valve member (16) to close the air supply channel (15, 115, 215) or the venting channel (14, 114, 214),
and with a pneumatic actuator (20) for actuating the switching valve member (16), which is pneumatically divided into an actuating chamber (21) and a return chamber (22) by a separating element (26) designed as membrane or bellows, wherein a first control pressure (p1) in the actuating chamber (21) acts against a spring return means and against a second control pressure (p2) in the return chamber (22),
**characterized in that**
the return chamber (22) comprises a return pressure channel (40) for providing compressed air to the return chamber (22) and a dynamic pressure channel (12, 112, 212) equipped with an outlet throttle (32) for discharging compressed air to a pressure sink, such as the atmosphere (atm).

2. Pneumatically actuated switching valve (1, 101, 201) according to claim 1, **characterized in that** the return pressure channel (40) is connected to the main air channel (13, 113, 213) and/or the air supply channel (15, 115, 215) and/or wherein the return pressure channel (40) connects the switching valve chamber (10) to the return chamber (22).

3. Pneumatically actuated switching valve (1, 101, 201) according to claim 1 or 2, **characterized in that** a control throttle (42) for setting the second control pressure (p2) is arranged in the return pressure channel (40).

4. Pneumatically actuated switching valve (1, 101, 201) according to claim 3, **characterized in that** the control throttle (42) has a variable throttle width, wherein in particular the control throttle (42) comprises a movable throttle member (44), such as a grub screw.

5. Pneumatically actuated switching valve (1, 101, 201) according to one of the preceding claims, **characterized in that** the spring return means is adjustable, wherein in particular a return spring (25, 125, 225) of the pneumatic actuator (20) comprises a setting means to set a lower minimum threshold member of the return force, which acts at least against the first control pressure (p1) in the actuating chamber (21).

6. Pneumatically actuated switching valve (1, 101, 201) according to one of the preceding claims, **characterized in that** the pneumatic actuator (20) and the switching valve chamber (10) are accommodated in the same housing (7), wherein in particular the housing (7) comprises at least or exactly two shell parts (72, 74), between which the switching valve member (16) and/or the separating element (26) is held.

7. Pneumatically actuated switching valve (1, 101, 201) according to claim 6, **characterized in that** the switching valve chamber (10) comprises an annular channel (17) at least partially, in particular completely, surrounding the switching valve member (16), which is in fluidic communication with the main air channel (13, 113, 213) and the air supply channel (15, 115, 215) and/or the venting channel (14, 114, 214), wherein in particular the return pressure channel (40) connects the return chamber (22) with the annular channel (17).

8. Pneumatically actuated switching valve (1, 101, 201) according to one of claims 6 or 7, **characterized in that** in the same outside connection surface (76) of the housing (7) a control pressure channel (11, 111, 211) for providing compressed air to the actuating chamber (21), the dynamic pressure channel (12, 112, 212), the main air channel (13, 113, 213), the venting channel (14, 114, 214) as well as the air supply channel (15, 115, 215) open, wherein in particular on the connection surface (76) is arranged a manipulator (46) for the movable throttle member (44).

9. Valve arrangement (100) comprising two pneumatically actuated switching valves (1, 101, 201), wherein at least one of the two pneumatically actuated switching valves (1, 101, 201) is designed according to one of claims 1 to 8, wherein the main air channel (13, 113) of the first switching valve (1, 101) is connected to a control pressure channel (11, 211) for providing compressed air to the actuating chamber (21) of the second switching valve (1, 201),
**characterized in that**
the main air channel (13, 113) of the first switching valve (1, 101) is connected via a self-actuating channel (160) to a control pressure channel (11, 111) for providing compressed air to the actuating chamber (21) of the first switching valve (1, 101).

10. Valve arrangement according to claim 9, **characterized in that** the self-actuating channel (160) comprises a pneumatic delay member, wherein in particular the delay member comprises a volume accumulator (164) and/or a self-actuating throttle (162).

11. Ventilation system (300) comprising a valve arrangement according to one of claims 9 or 10.

12. Ventilation system according to claim 11 for bellows ventilation, **characterized in that** the main air channel (13, 213) is connected to a pneumatic actuator (320) for bellows pressurization, wherein the valve arrangement (100) is configured to cyclically supply the pneumatic actuator (320) with compressed air.

## Revendications

1. Soupape de commutation (1, 101, 201) à actionnement pneumatique, pourvue d'une chambre (10) de soupape de commutation, dans laquelle débouchent trois canalisations d'air (13, 14, 15, 113, 114, 115, 213, 214, 215), les trois canalisations d'air (13, 14, 15, 113, 114, 115, 213, 214, 215) comprenant une canalisation d'alimentation d'air (15, 115, 215), destinée à recevoir de l'air comprimé à partir d'une source d'alimentation (70, 110, 310) d'air comprimé, une canalisation d'air (13, 113, 213) principale, destinée à aérer et à ventiler au moins un autre composant pneumatique et une canalisation de ventilation (14, 114, 214), destinée à restituer de l'air comprimé à un puits de pression, tel que l'atmosphère (atm),
dans la chambre (10) de soupape de commutation étant placé un élément (16) de soupape de commutation, pour fermer la canalisation d'alimentation d'air (15, 115, 215) ou la canalisation de ventilation (14, 114, 214), et pourvue d'un actionneur (20) pneumatique, destiné à actionner l'élément (16) de soupape de commutation, qui est pneumatiquement divisé par un élément de séparation (26) conçu sous la forme d'une membrane ou d'un soufflet en une chambre d'actionnement (21) et une chambre de rappel (22), une première pression de commande (p1) dans la chambre d'actionnement (21) agissant à l'encontre d'un rappel par ressort et à l'encontre d'une deuxième pression de commande (p2) dans la chambre de rappel (22),
**caractérisée en ce que**
la chambre de rappel (22) comporte une canalisation (40) de pression de rappel, destinée à mettre à disposition de l'air comprimé pour la chambre de rappel (22) et une canalisation (12, 112, 212) de pression dynamique équipée d'un étrangleur de sortie (32), destinée à restituer de l'air comprimé à un puits de pression, tel que l'atmosphère (atm).

2. Soupape de commutation (1, 101, 201) à actionnement pneumatique selon la revendication 1, **caractérisée en ce que** la canalisation (40) de pression de rappel est reliée avec la canalisation d'air (13, 113, 213) principale et / ou la canalisation d'alimentation d'air (15, 115, 215) et / ou la canalisation (40) de pression de rappel reliant la chambre (10) de soupape de commutation avec la chambre de rappel (22).

3. Soupape de commutation (1, 101, 201) à actionnement pneumatique selon la revendication 1 ou 2, **caractérisée en ce que** dans la canalisation (40) de pression de rappel est placé un étranglement de commande (42), destiné à régler la deuxième pression de commande (p2).

4. Soupape de commutation (1, 101, 201) à actionnement pneumatique selon la revendication 3, **caractérisée en ce que** l'étranglement de commande (42) présente une largeur d'étranglement variable, notamment l'étranglement de commande (42) comprenant un organe d'étranglement (44) mobile, tel qu'une vis sans tête.

5. Soupape de commutation (1, 101, 201) à actionnement pneumatique selon l'une quelconque des revendications précédentes, **caractérisée en ce que** le rappel par ressort est réglable, notamment un ressort de rappel (25, 125, 225) de l'actionneur pneumatique (20) comprenant un moyen de réglage, pour régler une valeur seuil minimale basse de la force de rappel, laquelle agit au moins à l'encontre de la première pression de commande (p1) dans la chambre d'actionnement (21).

6. Soupape de commutation (1, 101, 201) à actionnement pneumatique selon l'une quelconque des revendications précédentes, **caractérisée en ce que** l'actionneur pneumatique (20) et la chambre (10) de soupape de commutation sont logés dans le même boîtier (7), notamment le boîtier (7) comprenant au moins ou précisément deux parties de coque (72, 74), entre lesquelles est maintenu l'élément (16) de soupape de commutation et / ou l'élément de séparation (26).

7. Soupape de commutation (1, 101, 201) à actionnement pneumatique selon la revendication 6, **caractérisée en ce que** la chambre (10) de soupape de commutation comprend une canalisation annulaire (17) entourant sur au moins une partie de sa périphérie, notamment sur toute sa périphérie l'élément (16) de soupape de commutation, qui est en communication fluidique avec la canalisation d'air (13, 113, 213) principale et la canalisation d'alimentation d'air (15, 115, 215) et / ou la canalisation de ventilation (14, 114, 214), notamment la canalisation (40) de pression de rappel reliant la chambre de rappel (22) avec la canalisation annulaire (17).

8. Soupape de commutation (1, 101, 201) à actionnement pneumatique selon l'une quelconque des revendications 6 ou 7, **caractérisée en ce que** sur la même surface de raccordement (76) côté extérieur du boîtier (7) débouchent aussi bien une canalisation de pression de réglage (11, 111, 211), destinée à mettre à disposition de l'air comprimé pour la chambre d'actionnement (21), la canalisation (12, 112, 212) de pression dynamique, la canalisation d'air (13, 113, 213) principale, la canalisation de ventilation (14, 114, 214) ainsi qu'également la canalisation d'alimentation d'air (15, 115, 215), notamment sur la surface de raccordement (76) étant placé un manipulateur (46) pour l'organe d'étranglement (44) mobile.

9. Ensemble de soupapes (100), comprenant deux soupapes de commutation (1, 101, 201) à actionnement pneumatique, au moins l'une des deux soupapes de commutation (1, 101, 201) à actionnement pneumatique étant réalisée selon l'une quelconque des revendications 1 à 8, la canalisation d'air (13, 113) principale de la première soupape de commutation (1, 101) étant réalisée avec une canalisation de pression de réglage (11, 211) destinée à mettre à disposition de l'air comprimé pour la chambre d'actionnement (21) de la deuxième soupape de commutation (1, 201),
**caractérisé en ce que**
la canalisation d'air (13, 113) principale de la première soupape de commutation (1, 101) est reliée par l'intermédiaire d'une canalisation d'auto-actionnement (160) avec une canalisation de pression de réglage (11, 111), destinée à mettre à disposition de l'air comprimé pour la chambre d'actionnement (21) de la première soupape de commutation (1, 101).

10. Ensemble de soupapes selon la revendication 9, **caractérisée en ce que** la canalisation d'auto-actionnement (160) comprend un élément de temporisation pneumatique, notamment l'élément de temporisation comprenant un accumulateur volumétrique (164) et / ou un étranglement à auto-actionnement (162).

11. Système de respirateur (300) comprenant un ensemble de soupapes selon l'une quelconque des revendications 9 ou 10.

12. Système de respirateur selon la revendication 11 pour la ventilation au soufflet, **caractérisé en ce que** la canalisation d'air (13, 213) principale est reliée avec un actionneur (320) pneumatique, destiné à contraindre le soufflet, l'ensemble de soupapes (100) étant aménagé pour alimenter cycliquement l'actionneur (320) pneumatique avec de l'air comprimé.
